(19) Europäisches Patentamt
European Patent Office
Office européen des brevets

(11) EP 1 067 901 B1

(12) EUROPEAN PATENT SPECIFICATION

(45) Date of publication and mention of the grant of the patent:
**02.01.2003 Bulletin 2003/01**

(21) Application number: **99916244.9**

(22) Date of filing: **31.03.1999**

(51) Int Cl.[7]: **A61K 7/48**, A61K 7/50, A61K 7/06, A61K 7/32, A61K 7/34, A61K 7/38

(86) International application number:
**PCT/US99/07134**

(87) International publication number:
**WO 99/051192 (14.10.1999 Gazette 1999/41)**

# (54) LOW RESIDUE COSMETIC COMPOSITION

RÜCKSTANDSARME KOSMETISCHE ZUSAMMENSETZUNG

COMPOSITION COSMETIQUE FAIBLEMENT RESIDUELLE AMELIOREE

(84) Designated Contracting States:
**AT BE CH CY DE DK ES FI FR GB GR IE IT LI NL PT SE**
Designated Extension States:
**RO**

(30) Priority: **03.04.1998 US 54666**
**19.03.1999 US 273152**

(43) Date of publication of application:
**17.01.2001 Bulletin 2001/03**

(60) Divisional application:
**02075748.0 / 1 224 928**

(73) Proprietor: **Colgate-Palmolive Company**
**New York, N.Y. 10022 (US)**

(72) Inventors:
- **POTECHIN, Kathy, J.**
  **Short Hills, NJ 07078 (US)**
- **GUENIN, Eric, P.**
  **Pennington, NJ 08534 (US)**
- **TANG, Xiaozhong**
  **Bridgewater,NJ 08807 (US)**
- **MATTAI, Jairajh**
  **Piscataway, NJ 08855 (US)**
- **LINN, Elizabeth**
  **Lyndhurst, NJ 07071 (US)**
- **LEE, Wilson**
  **Bloomfield, NJ 07003 (US)**
- **VINCENTI, Paul**
  **Jefferson, NJ 07849 (US)**

(74) Representative: **Prins, Adrianus Willem et al**
**Vereenigde,**
**Nieuwe Parklaan 97**
**2587 BN Den Haag (NL)**

(56) References cited:
**EP-A- 0 381 318**
**EP-A- 0 818 190**
**EP-A- 0 908 175**
**EP-A- 0 940 923**
**WO-A-96/18374**
**WO-A-98/00102**
**WO-A-98/35649**
**WO-A-99/00108**
**WO-A-99/32070**

- **DATABASE "CHEMICAL ABSTRACTS" (HOST: STN, Karlsruhe, DE); abstract 123: 179 127; & JP 07 165 529 A (KOSEI KK) 27 JUNE 1995 XP002115983**
- **DATABASE "CHEMICAL ABSTRACTS" (HOST: STN Karlsruhe, DE); abstract 131:106 625; & JP 11 193 214 A (KOSEI KK) 21 JULY 1999 XP002115984**
- **DATABASE "CHEMICAL ABSTRACTS" (HOST: STN Karlsruhe, DE); abstacts 126: 108 664; & JP 08 295 620 A ( KANEBO, Ltd) 12 NOVEMBER 1996 XP002115985**
- **DATABASE "CHEMICAL ABSTRACTS" (HOST: STN, Karlsruhe, DE); abstract 128: 26 749; & JP 09 286 710 A (SHISEIDO CO., Ltd) 4 NOVEMBER 1997 XP002115986**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 067 901 B1

## Description

Background of the Invention

The present invention is directed to a cosmetic antiperspirant and/or deodorant composition (especially a gelled composition) which leaves little or no visible (white) residue on the skin, and which has improved properties especially in the areas of glidability, skin feel, smoothness, reduction in tack, reduction in syneresis, and increased efficacy. In particular, the present invention may be used to formulate cosmetic antiperspirant and/or deodorant compositions which contain at least one antiperspirant active material, which leaves little or no visible residue on the skin, and which has improved aesthetic properties as described above. A particular area of emphasis is underarm antiperspirant and/or deodorant compositions which utilize an antiperspirant active and optionally an antimicrobial.

There is a continuing trend to develop new and superior cosmetic compositions especially for the reduction and/or elimination of wetness and/or odor under the arms. Particular efforts include developing clearer and lower residue products as well as raising the performance and aesthetics of such products. Various product forms have included sticks (especially gel/sticks), gels. soft solids, roll-ons. aerosols and creams. Of these various forms the sticks, gels, soft solids creams and roll-ons are made with a liquid base material incorporating a solidifying agent and/or gelling agent and/or thickening agent. Generally, these forms include a solution of the cosmetically active ingredient in a suitable vehicle, a suspension of the active ingredient in a carrier vehicle, or a multiphasic dispersion or emulsion in which a solution of the active ingredient is dispersed or suspended in some continuous phase or in which the solubilized active ingredient constitutes the continuous phase.

A variety of soft-solid formulations are known. These include formulations made with the following ingredients:

(a) clay thickening agent and an activator for the clay: for example, U.S. Patent Number 5,019,375 to Tanner et al; and U.S. Patent Number 4,526,780 to Marschner et al;
(b) particulate thickening agents such as fumed silica: for example, US-A-5,069,897; and US-A-4,937,069;
(c) selected volatile and/or non-volatile alkylmethylsiloxanes such as those including a structuring wax: for example, US-A-5,235,188; and WO 97/16161 and WO 97/16162; and
(d) triglyceride gellants such as the glyceryl tribehenate described in US-A-5,718,890.

An attempt to use silicone gel materials without the incorporation of clay or wax into the cosmetic formulations is described in WO 97/44010. One type of silicone gel material in this application is made by combining (a) a volatile siiicone material and (b) an organopolysiloxane material (or silicone elastomer) as a gelling agent wherein the organopolysiloxane material (silicone elastomer) can be a reaction product of a vinyl-terminated siloxane polymer and a silicon hydride cross-linking agent. Related technology is also disclosed in WO 98/00097, WO 98/00104 and WO 98/00105 on cross-linked non-emulsifying elastomers.

US-A-5,599,533 and WO-A-96/18374 describe a stable water-in-oil emulsion system formed with an organopolysiloxane elastomer, a vehicle in which the elastomer is dispersed or dispersible, a stabilizing agent, a surfactant and an aqueous component. A commercial product known as "REVELATION™" retexturizing complex for hands and chest sold by the same assignee contains a silicone gel material with an organopolysiloxane component and octamethylcyclotetrasiloxane.

Other examples of discussion of elastomer type materials and/ or methods for processing such materials may be found in WO 98/00097; WO 98/00104; WO 98/00105; WO 98/18438; and WO 98/42307.

EP 0 787 758 A1 teaches a method for solvent thickening by using a silicone latex having a plurality of crosslinked polysiloxane particles.

WO 96/06594 describes a silicone-free surfactant or silicone-flee surfactant blend having an HLB value of 0.1 to about 10. an organic phase comprising a volatile silicone compound or a volatile hydrocarbon compound, and water.

Borate cross-linkers useful in topically active compositions are described in EP 0 676 193 A2.

These types of compositions are not totally satisfactory in cosmetic applications, especially underarm applications since they tend to synerese, form films, etc. Thus, there remains a need for improved formulations which exhibit improved properties and performance, especially in the area of aesthetics and efficacy.

In particular, one of the major problems with elastomer-containing formulations is the formation of a film after application to the surface of the skin. The hydrophobic nature of such a film may prevent an active ingredient (for example, an antiperspirant active) from reaching the targeted site (for example, when applied to the underarm area for antiperspirant/deodorant purposes).

Thus, it is an object of the present invention to provide cosmetic compositions which use selected silicone gel material to form compositions which leave little or no visible (white) residue on the skin, and which have improved properties especially in the areas of glidability, skin feel, smoothness, reduction in tack, reduced syneresis and increased efficacy.

It is another object of the invention to provide cosmetic compositions which reduce or eliminate the tendency to form films on the skin when applied in use. which might affect efficacy.

It is a further object of the invention to provide improved cosmetic compositions with the improvements as previously described and which are useful as antiperspirants and/or deodorants.

It is yet another object of this invention to provide cosmetic compositions which exhibit a reduction or elimination in the formation of films when applied to the skin surface.

It is still another object of the invention to provide underarm products containing an active ingredient which are made with a selected surfactant and which exhibit a reduction or elimination in the formation of films when applied to the skin surface with an increase in the efficacy of the antiperspirant active.

These and other objects of the invention will be apparent from the following description of the invention.

The following four documents are documents citable under Art 54(3) EPC.

EP-A-0 940 423 describes emulsions of silicones with non-aqueous hydroxylic solvents.

According to the working examples a similar silicone material as used in accordance with the present invention is used together with a nonionic surfactant and an antiperspirant active.

In WO-A-98/35649 sun screen compositions are described which may contain a preservative and a silicone material, similar to the one used in the present invention. Ester emollients and nonionic surfactants are also incorporated in the compositions described.

EP-A-0 908 175 discloses topical compositions containing a solid elastomeric organopolysiloxane and spherical particles. The compositions can comprise non-ionics and ester emollients and cosmetically active compounds.

WO 99/00108 describes compositions requiring ferulic acid or the $C_{1-30}$ alcohol esters thereof. It may contain a similar silicone gel material as used in the present invention.

## Summary of the Invention

This invention comprises low residue cosmetic antiperspirant and/or deodorant compositions (especiall underarm products) which are made by combining at least one antiperspirant active material; a silicone gel material itself comprising an elastomer composition; at least one selected surfactant having an HLB value in the range of 8-16; and at least one particular ester-type emollient material. The compositions of this invention exhibit reduced or eliminated film formation when applied to the skin and increased availability of the active ingredient.

## Detailed Description of the Invention

The cosmetic compositions of this invention can be made in the form of solids (for example, sticks (especially gel/sticks), gels, soft solids and creams) or liquids which have a viscosity of at least 1.000 mPa.s (centiposie ("cps")).

The compositions of the present invention are made by combining:

a) at least one antiperspirant active material;

(b) a silicone gel material which is made by combining:

  (i) a cross-linked organopolysiloxane material as a gelling (solidifying) agent wherein the organopolysiloxane is made from a member selected from the group consisting of at least one silicone hydride cross-linking agent and at least one member selected from the group consisting of (A) a vinyl-terminated polysiloxane and (B) an alpha, omega diene; and

  (ii) a liquid base vehicle for the gelling agent wherein the vehicle is made by combining at least one member of the group consisting of volatile silicone materials and non-volatile silicone materials, and optionally wherein at least one liquid emollient which is soluble or miscible in the selected silicone material(s) is added to form the vehicle (in this role the emollient is also referred to as "emollient material");

(c) a nonionic surfactant having an HLB value of 8-16, particularly 8-12; and

(d) at least one emollient material selected from the group consisting of isopropyl myristate, isopropyl palmitate, isopropyl stearate, isopropyl isostearate, butyl stearate, octyl stearate, hexyl laurate, cetyl stearate, diisopropyl adipate, isodecyl oleate, diisopropyl sebacate, isostearyl lactate, $C_{12-15}$ alkyl benzoate, myreth-3 myristate, dioctyl malate, neopentyl glycol diheptanoate, neopentyl glycol dioctanoate, dipropylene glycol dibenzoate, $C_{12-15}$ alcohols lactate, isohexyl decanoate, isohexyl caprate, diethylene glycol dioctanoate, octyl isononanoate, isodecyl octanoate, diethylene glycol diiaononartoate, isononyl isononanoate, isostearyl isostearate, behenyl behenate, $C_{12-15}$ alkyl fumarate, laureth-2-benzoate, propylene glycol isoceteth-3 acetate, propylene glycol ceteth-3 acetate, octyldodecyl myristate, cetyl recinoleate, and myristyl myristate.

The cosmetical antiperspirant active material can optionally be combined with deodorant active materials, insect repellents, antifungal agents, antibacterials agents, and fragrances. Formulations of particular interest are antiperspirants.

As antiperspirant active, any of the known antiperspirant active materials can be utilized. These include, by way of example (and not of a limiting nature), aluminum chlorohydrate, aluminum chloride, aluminum sesquichlorohydrate, zirconyl hydroxychloride, aluminum-zirconium glycine complex (for example, aluminum zirconium trichlorohydrex gly, aluminum zirconium pentachlorohydrex gly, aluminum zirconium tetrachlorohydrex gly and aluminum zirconium octochlorohydrex gly), aluminum chlorohydrex PG, aluminum chlorohydrex PEG, aluminum dichlorohydrex PG. and aluminum dichlorohydrex PEG. The aluminum-containing materials can be commonly referred to as antiperspirant active aluminum salts. Generally, the foregoing metal antiperspirant active materials are antiperspirant active metal salts. In the embodiments which are antiperspirant compositions according to the present invention, such compositions need not include aluminum-containing metal salts, and can include other antiperspirant active materials, including other antiperspirant active metal salts. Generally, Category I active antiperspirant ingredients listed in the Food and Drug Administration's Monograph on antiperspirant drugs for over-the-counter human use can be used. In addition, any new drug, not listed in the Monograph, such as aluminum nitratohydrate and its combination with zirconyl hydroxychlorides and nitrides, or aluminum-stannous chlorohydrates, can be incorporated as an antiperspirant active ingredient in antiperspirant compositions according to the present invention.

Antiperspirant actives can be incorporated into compositions according to the present invention in amounts in the range of 0.1 - 30%, preferably 15 - 25%, by weight, of the total weight of the composition. The amount used will depend on the formulation of the composition. For example, at amounts in the lower end of the broader range (for example, 0.1 - 10%), the antiperspirant active material will not substantially reduce the flow of perspiration, but will reduce malodor, for example, by acting as an antimicrobial material.

The antiperspirant active material is desirably included as particulate matter suspended in the composition of the present invention in amounts as described above, but can also be added as solutions or added directly to the mixture.

Additionally, a deodorant fragrance may be used in an amount of 0.05-5.0% by weight based on the total weight of the composition.

An antimicrobial agent, for example bacteriostatic quaternary ammonium compounds such as 2-amino-2-methyl-1-propanol (AMP), cetyltrimethylammonium bromide, cetyl pyridinium chloride, 2, 4, 4'-trichloro-2'-hydroxydiphenylether (Triclosan), N-(4-chlorophenyl)-N'-(3,4-dichlorophenyl)urea (Triclocarban), silver halides, octoxyglycerin (Sensiva™ SC 50) and various zinc salts (for example, zinc ricinoleate) may also be included in formulations of the present invention. The bacteriostat can, illustratively, be included in the composition in an amount of 0.01-1.0% by weight, of the total weight of the composition. Triclosan, can illustratively be included in an amount of from 0.05% to about 0.5% by weight, of the total weight of the composition.

The silicone gel material includes at least one crosslinked organopolysiloxane material as a gelling agent and a vehicle as described herein. Each organopolysiloxane is made from a cross-linking agent and at least one member selected from the group consisting of siloxanes containing at least one vinyl group (hereinafter referred to as a "vinyl polysiloxane") and alpha omega dienes. Suitable vinyl polysiloxanes include:

(a) vinyl terminated polysiloxanes such as that of Formula I:

$$CH_2=CH\text{-}Si(CH_3)_2\text{-}O\text{-}(\text{-}Si(CH_3)_2\text{-}O\text{-})_n\text{-}Si(CH_3)_2\text{-}CH=CH_2 \qquad \text{Formula I}$$

(b) vinyl functional copolymers such as that of Formula II:

$$(CH_3)_3Si\ \text{-}O\text{-}(Si(CH_3)_2\text{-}O\text{-})_m\text{-}(Si(CH_3)(CH=CH_2)),\text{-}Si\ (CH_3)_3 \qquad \text{Formula II}$$

where n = a number from 1-100, particularly 10-50; and
m= a number from 1-100, particularly 10-50.

Particular examples of vinyl polysiloxanes include, but are not limited to:

(a) polydimethylsiloxane, which is monovinyl terminated;
(b) vinylmethyl, dimethylsiloxane copolymer which is trimethylsiloxy terminated;
(c) vinylmethyl, dimethylsiloxane copolymer which is vinyl dimethyl terminated;
(d) divinylmethyl terminated polydimethyl siloxanes;

(e) vinyl Q-resin
(f) vinylphenylmethyl terminated dimethyl siloxanes;
(g) cyclic vinylmethyl dimethyl siloxanes;
(h) T-structure polydimethyl siloxanes with vinyl at branchpoint;
(i) T-structure polydimethyl siloxane with vinyl at branch terminus;
(j) diphenyl dimethyl copolymer which is vinyl terminated;
(k) vinyl terminated polydimethyl siloxanes;
(l) vinyl terminated trifluoropropyl methyl siloxane - dimethylsiloxane copolymer;
(m) vinyl terminated diethyl siloxane copolymer;
(n) vinyl methyl siloxane - dimethyl siloxane copolymer which is trimethylsiloxy terminated
(o) vinyl gums;
(p) vinyl methyl siloxane homopolymers; arid
(q) mixtures of two or more of the foregoing.

Suitable alpha, omega dienes include those described in US-A-5,880,210, especially those of Formula:

$$CH_2=CH(CH_2)_xCH=CH_2,$$

where x is a number in the range of 1-20. Particular examples of suitable alpha, omega dienes include: 1,4-pentadiene; 1,5-hexadiene: 1,6-heptadiene: 1,7-octadiene; 1,8-nonadiene; 1,11-dodecadiene; 1,13-tetradecadiene; and 1,19-eicosadiene.

Suitable crosslinking agents include hydride terminated polydimethylsiloxanes of Formula III:

$$H\text{-}Si\,(CH_3)_2\text{-}O\text{-}(Si(CH_3)_2\text{-}O\text{-})_p\text{-}Si(CH_3)_2\text{-}H \qquad \text{Formula III}$$

where p= a number from 1-50, particularly 5-20.

Particular examples of cross-linking agents are hydride functional polymers (≡ SiH). Typical hybrid crosslinking agents are methylhydro-dimethylsiloxane copolymer with 20-60% methyl hydrogen. In selected circumstances hydride terminated siloxanes may be used for chain extension. Examples of suitable crosslinking agents include but are not limited to:

(a) methylhydrosiloxane - dimethylsiloxane copolymer,
(b) polymethylhydrosiloxanes;
(c) polyethylhydrosilane;
(d) polyphenyl - (dimethylhydrosiloxy)siloxane which is hydride terminated;
(e) methylhydrosiloxane - phenylmethylsiloxane copolymer which is hydride terminated; and
(f) methylhydrosiloxane - octylmethylsiloxane copolymer.

The reaction between a vinyl polysiloxane and a cross-linking agent may be accomplished in several ways:

(a) hydrosilylation addition reaction - This reaction relies on the ability of the hydrosilane bond (=SiH) to add across a carbon-carbon double bond in the presence of noble metal catalysts, particularly platinum;

$$\equiv SiH + CH_2=CHSi\equiv \rightarrow \equiv SiCH_2CH_2Si\equiv$$

(b) peroxide induced free radical reactions - These reactions are an adaptation of conventional organic chemistry and relies on the availability of C-H bonds (already present in the methyl groups of the polydimethylsiloxane) to effect the cross-linking. These reactions use peroxide or radiation to initiate cross-linking. The peroxide-induced cross-linking can be achieved by using two categories of peroxide, the so-called vinyl specific catalysts, such as aryloxy peroxides, which do not need the vinyl groups to effect cross-linking. In the case of vinyl specific peroxides the level of vinyl functionality needed to achieve the desired level of cross-linking is typically in the range of 0.1-0.15% weight/weight of the polymer. For polydimethylsiloxane and diaryloxyperoxides the cure mechanism proceeds by hydrogen abstraction from the $\equiv Si\text{-}CH_3$, by the peroxy radical, followed by combination of the resulting $\equiv Si\dot{C}H_2$ radicals formed to give a $\equiv SiCH_2CH_2Si\equiv$ cross-link:

$$ArOOAr \rightarrow 2\ Ar\dot{O}$$

$$Ar\dot{O} + CH_3Si\equiv \rightarrow ArOH + \dot{C}H_2Si\equiv$$

$$\equiv Si\dot{C}H_2 + \dot{C}H_2Si\equiv \rightarrow \equiv SiCH_2CH_2Si\equiv$$

(c) condensation reactions - these reactions rely on the ability of some organic groups (such as alkoxy or acyloxy) attached to silicon (=SiOR) to react with water to produce silanol (=SiOH groups) which can then further react with the starting materials or another silanol group to produce a siloxane cross-link:

$$\equiv SiOR + HOH \rightarrow \equiv SiOH + ROH$$

then

$$\equiv SiOR + \equiv SiOH \rightarrow \equiv SiOSi\equiv + ROH$$

or

$$\equiv SiOH + \equiv SiOH \rightarrow \equiv SiOSi\equiv + HOH$$

and

(d) hydridosilane/silanol reactions - Hydridosilane groups can react in a similar way as was described for condensation reactions; the reaction can be represented as either a one-step or two-step process:

$$\equiv SiH + \equiv SiOH \rightarrow \equiv SiOSi\equiv + H_2$$

or

$$\equiv SiH + H_2O \rightarrow \equiv SiOH + H_2$$

$$\equiv SiOH + \equiv SiOH \rightarrow \equiv SiOSi\equiv + H_2O$$

In one particular example, a silicone gel as described in US-A-5,654,362 is made by reacting an ≡Si-H containing polysiloxane with an alpha, omega-diene. The reaction is conducted in the presence of a platinum catalyst and in the presence of a low molecular weight silicone oil. The reaction is continued until a gel is formed by cross-linking and addition of ≡Si-H across double bonds in the alpha, omega-diene ($CH_2$=$CH(CH_2)_xCH$=$CH_2$, where x = 1-20). Examples herein are 1,4-pentadiene; 1,5-hexadiene; 1,6-heptadiene; 1,7-octadiene; 1,8-nonadiene; 1,9-decadiene; 1,11-dodecadiene; 1,13-tetradecadiene; and 1,19-eicosadiene.

Particular examples of suitable elastomers are SFE 167™, a cetearyl dimethicone/vinyl dimethicone crosspolymer from GE Silicones (Waterford, N.Y.); SFE 168™, a cyclomethicone (and) dimethicone/vinyl dimethicone crosspolymer from GE Silicones; vinyl dimethicone crosspolymers such as those available from Shin Etsu Silicones of America (Akron, Ohio) under trade names KSG-15 (cyclomethicone (and) dimethicone/vinyl dimethicone crosspolymer), KSG-16 (dimethicone (and) dimethicone/vinyl dimethicone crosspolymer), KSG-17 (cyclomethicone (and) dimethicone/vinyl dimethicone crosspolymer), KSG-18 (phenyl trimethicone (and) dimethicone/phenyl vinyl dimethicone crosspolymer); and KSG-20 (dimethicone copolyol crosspolymer; dimethicone/vinyl dimethicone crosspolymer from Dow Coming Corporation (Midland, MI) under trade name Dow Corning 9506 Cosmetic Powder; and a mixture of cyclomethicone and stearyl-vinyl/hydromethylsiloxane copolymer available from Grant Industries, Inc. (Elmwood Park, NJ) under the trade name Gransil SR-CYC.

As described above, a liquid base vehicle for the gelling agent is used wherein the vehicle is made by combining at least one member of the group consisting of volatile silicone materials and non-volatile silicone materials, and optionally wherein at least one liquid emollient which is soluble or miscible in the selected silicone material(s) is added to form the vehicle.

The silicone materials used in providing the silicone gel material for forming the composition of the present invention may be selected from the group consisting of conventional cyclic and linear volatile and non-volatile silicones which act as a swelling agent for the suitable elastomer. Illustratively. and not by way of limitation, the volatile silicones are one or more members selected from the group consisting of cyclic polydimethylsiloxanes such as those represented by Formula IV:

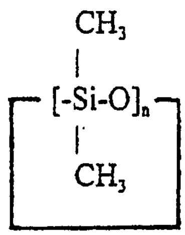

Formula IV

where n is an integer with a value of 3-7, particularly 5-6. By volatile silicone material is meant a material that has a measurable vapor pressure at ambient temperature. For example, DC-345™ fluid from Dow Corning Corporation (Midland, Michigan) is a type of cyclomethicone which can be used. These include a tetramer (or octylmethylcyclotetrasiloxane) and a pentamer (or decamethylcyclo-pentasiloxane). The nonvolatile and volatile linear silicones are one or more members selected from the group consisting of linear polydimethylsiloxanes such as those represented by Formula V:

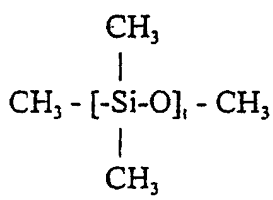

Formula V

and t is selected so that the molecular weight ranges from 800-260,000 and the viscosity ranges from 0.05-6000 $cm^2/s$ (5-600,000 centistokes), for example Dimethicone DC 200™ from Dow Corning.

The vehicle may also be formed with at least one additional component selected from the group consisting of liquid emollients which are soluble or miscible in the silicone material(s) used to make the vehicle. By adding the additional liquid emollient to form the vehicle, such liquid emollient is characterized herein as part of the vehicle and not as part of the optional emollient additive which optional emollient additive may be added after the gel is formed. Liquid emollients suitable for use in forming a vehicle for this invention include any of those materials listed below under the full description of emollients provided they are liquids at room temperature and are soluble or miscible in the silicone materials selected to form the vehicle. Particular examples of such emollients useful in forming the vehicles for this invention include, but are not limited to:

(a) hydrocarbons - especially hydrogenated polyisobutene;
(b) esters - especially diisopropyl adipate, $C_{12-15}$ alkyl benzoates and neopentyl glycol diheptanoate; and
(c) silicones and silanes - especially phenyl trimethicone and dimethicone.

Various techniques may be used to make gel materials from the elastomers listed above. These techniques include mechanical processes with high shear and/or high pressure for example, homogenization or sonolation (for example as described in WO 98/00104, and US-A-5,854,336).

Silicone gel materials made from the elastomers described above are available from GE Silicones (Waterford, NY) as GE 839™, Grant Industries, Inc. (Elmwood Park, NJ) as Gransil GCM™, and Dow Corning Corporation (Midland, Michigan) as DC 9040™. The elastomers used in this invention may be used in various concentrations, particle size distributions, viscosities and rheological profiles.

It is preferred that the silicone gel materials of the present invention have a select rheology profile. The rheology profile is defined herein in terms of the storage modulus (G'), the loss modulus (G") and the G"/G' ratio. The storage modulus represents how elastic or structured a sample is, the loss modulus represents how viscous a sample is. The numerical ratio G"/G' represents the extent to which the silicone gel materials exhibit certain viscous and elastic characteristics. Measurements for G' and G" for purposes of defining the silicone gel materials of the present invention may be determined at ambient conditions using conventional techniques known to those skilled in the art. For example, a $CSL^2$ 500 Controlled Stress Rheometer™, available from TA Instruments, New Castle, Delaware, can be used with a plate configuration. A sample size of about 5 cubic centimeters of the silicone gel material to be evaluated is carefully sampled with minimal application of shear force and is placed between the plate fixtures on the rheometer for measurements of G' and G" in Pascals. The G"/G' ratio is then determined. The silicone gel materials should have a G"/G' ratio from about 0.001-50, preferably from 0.005-20, and more preferably, from 0.01-10. Compositions formulated with silicone gel materials with the above rheology profile exhibit improved product attributes and/or performance such as reduced syneresis. better spreadability, less balling, smoother application and better structure.

In the final cosmetic composition the silicone gel material can be included in the cosmetic composition in an amount of 10-80% by weight based on the total weight of the final cosmetic composition.

The surfactants useful in this invention have an HLB (hydrophilic-lipophilic balance) value of 8-16 (more particularly 8-12). The HLB parameter is a well known parameter the calculation of which is disclosed and explained in numerous references. This HLB value can be determined using the traditional HLB system, the Three Dimensional HLB system (which is tailored for silicones) or the Cohesive Energy Ratio theory based on solubility parameters as described in Griffin, W.C., J. Soc. Cosmetic Chemists. 1:311, (1949); O'Lenick, Jr., A.J., et al. Cosmetics & Toiletries, Vol. 3:37-44 (October 1996); Beenbower, A. et al, McCutcheon's Detergents and Emulsifiers, The Manufacturing Confectioner Publishing Co., Glen Rock, New Jersey (1971) at pages 223-235; Fedors, R.F., Polymer Engineering and Science, Vol. 4, No.2 at pages 147-154 (February 1974). Any of these systems may be used to determine hydrophilicity and/or lipophilicity of a surfactant, although some variations may be obtained in HLB values. In particular, for nonionic surfactants, data obtained by actual analysis is usually a more accurate measure of HLB values (rather than theoretical determinations). For purposes of this invention it is intended that either the actual or theoretical HLB value may be used as the basis for selection.

With regard to nonionic surfactants, those used in this invention (which can also be a mixture or blend of surfactants) include, but are not limited to at least one member selected from the group consisting of:

(a) sorbitan esters and ethoxylated sorbitan esters (for example PEG-20 sorbitan isostearate, sorbitan monolaurate, polysorbate-40, polysorbate-60, polysorbate-80 and polysorbate-120);
(b) ethoxylates (for example, Ceteth-20, PEG-30 castor oil, PEG-40 hydrogenated castor oil. PEG-60 hydrogenated castor oil, Laureth-7, Isolaureth-6, Steareth-10. Steareth-20, Steareth-21, Steareth-100, Ceteareth-12, Oleth-5, Oleth-10, Ceteareth-20, Oleth-9, Oleth-20 and PPG-3-Buteth-5);
(c) ethoxylated adducts (for example, PEG-25 stearate, glyceryl stearate and PEG-100 stearate);
(e) propoxylates (for example, PPG-10 butanediol, PPG-1-PEG-9-lauryl glycol ether, PPG-3-deceth-3, PPG-5-ceteth-20);
(f) ethoxylated modified triglycerides (for example, PEG-20 corn glycerides, PEG-12 palm kernel glycerides);
(g) alkylphenol aromatic ethoxylates (for example, dinonylphenol ethoxylate with 9 moles of EO, octylphenol ethoxylate with 20 moles of EO, octylphenol ethoxylate with 40 moles of EO);
(h) block copolymers which are alkoxylated glycols having ethoxylated and propoxylated segments (for example. Poloxamers 182™ and 234™, Poloxamer 105 Benzoate™, and Meroxapol 174™);
(i) silicone polyethers (for example, (1) dimethicone copolyols (SIL WET™ L-7087, L-7200 and L-7657); (2) dimethicone copolyol laurate (SILWAX™ WS-L); (3) dimethicone copolyol isostearate (SILWAX WS-IS™); (4) polydimethicone copolyol (SILSOFT 477™); (5) Eugenodimethicone copolyol (SILSOFT Shine™); wherein the nonionic surfactant is selected so that it has an HLB (hydrophilic-lipophilic balance) value of 8-16 (more particularly 8-12).

The surfactant or blend of surfactants incorporated into the compositions of the present invention can, illustratively, be included in amounts of 0.1-20%, preferably 0.5-10%, and more preferably 1-5%, by weight based on the total weight of the composition.

The compositions of the present invention can also include other optional ingredients to improve the aesthetics and/or performance of the cosmetic compositions of the invention. These include emollients, thickeners, colorants, fillers, fragrances, masking agents, etc.

Emollients are a known class of materials in this art, imparting a soothing effect to the skin. These are ingredients which help to maintain the soft, smooth, and pliable appearance of the skin. Emollients are also known to reduce whitening on the skin and/or improve aesthetics. Examples of chemical classes from which suitable emollients can be found include:

(a) fats and oils which are the glyceryl esters of fatty acids, or triglycerides. normally found in animal and plant tissues, including those which have been hydrogenated to reduce or eliminate unsaturation. Also included are synthetically prepared esters of glycerin and fatty acids. Isolated and purified fatty acids can be esterified with glycerin to yield mono-, di-, and triglycerides. These are relatively pure fats which differ only slightly from the fats and oils found in nature. The general structure may be represented by Formula VI:

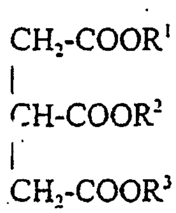

Formula VI

wherein each of $R^1$, $R^2$, and $R^3$ may be the same or different and have a carbon chain length (saturated or unsaturated) of 7 to 30. Specific examples include peanut oil, sesame oil, avocado oil, coconut, cocoa butter, almond oil, safflower oil, corn oil, cotton seed oil, castor oil, hydrogenated castor oil, olive oil, jojoba oil, cod liver oil, palm oil. soybean oil, wheat germ oil, linseed oil, and sunflower seed oil;

(b) hydrocarbons which are a group of compounds containing only carbon and hydrogen. These are derived from petrochemicals. Their structures can vary widely and include aliphatic, alicyclic and aromatic compounds. Specific examples include paraffin, petrolatum, hydrogenated polyisobutene, and mineral oil.

(c) esters which chemically, are the covalent compounds formed between acids and alcohols. Esters can be formed from almost all acids (carboxylic and inorganic) and any alcohol. Esters here are derived from carboxylic acids and an alcohol. The general structure would be $R^4CO\text{-}OR^5$. The chain length for $R^4$ and $R^5$ can vary from 7 to 30 and can be saturated or unsaturated, straight chained or branched. Specific examples include isopropyl myristate, isopropyl palmitate, isopropyl stearate, isopropyl isostearate, butyl stearate, octyl stearate, hexyl laurate, cetyl stearate, diisopropyl adipate, isodecyl oleate, diisopropyl sebacate, isostearyl lactate, $C_{12-15}$ alkyl benzoates, myreth-3 myristate, dioctyl malate, neopentyl glycol diheptanoate, neopentyl glycol dioctanoate, dipropylene glycol dibenzoate. $C_{12-15}$ alcohols lactate, isohexyl decanoate, isohexyl caprate, diethylene glycol dioctanoate, octyl isononanoate, isodecyl octanoate, diethylene glycol diisononanoate, isononyl isononanoate, isostearyl isostearate, behenyl behenate, $C_{12-15}$ alkyl fumarate, laureth-2 benzoate, propylene glycol isoceteth-3 acetate, propylene glycol ceteth-3 acetate, octyldodecyl myristate, cetyl ricinoleate, myristyl myristate.

(d) saturated and unsaturated fatty acids which are the carboxylic acids obtained by hydrolysis of animal or vegetable fats and oils. These have general structure $R^6COOH$ with the $R^6$ group having a carbon chain length between 7 and 30 , straight chain or branched. Specific examples include lauric, myristic, palmitic, stearic, oleic, linoleic and behenic acid.

(e) saturated and unsaturated fatty alcohols (including guerbet alcohols) with general structure $R^7OH$ where $R^7$ can be straight or branched and have carbon length of 7 to 30. Specific examples include lauryl, myristyl, cetyl, isocetyl, stearyl, isostearyl, oleyl, ricinoleyl and erucyl alcohol;

(f) lanolin and its derivatives which are a complex esterified mixture of high molecular weight esters of (hydroxylated) fatty acids with aliphatic and alicyclic alcohols and sterols. General structures would include $R^8CH_2\text{-}(OCH_2CH_2)_nOH$ where $R^8$ represents the fatty groups derived from lanolin and n=5 to 75 or $R^9CO\text{-}(OCH_2CH_2)_nOH$ where $R^9CO$- represents the fatty acids derived from lanolin and n=5 to 100. Specific examples include lanolin, lanolin oil, lanolin wax, lanolin alcohols, lanolin fatty acids, isopropyl lanolate, ethoxylated lanolin and acetylated lanolin alcohols.

(g) alkoxylated alcohols wherein the alcohol portion is selected from aliphatic alcohols having 2-18 and more particularly 4-18 carbons, and the alkylene portion is selected from the group consisting of ethylene oxide, and propylene oxide having a number of alkylene oxide units from 2-53 and, more particularly, from 2-15. Specific examples include PPG-14 butyl ether and PPG-53 butyl ether.

(h) silicones and silanes the linear organo-substituted polysiloxanes which are polymers of silicon/oxygen with general structure:

(i) $(R^{10})_3SiO(Si\ (R^{11})_2O)_xSi(R^{12})_3$ where $R^{10}$, $R^{11}$ and $R^{12}$ can be the same or different and are each independently selected from the group consisting of phenyl and C1-C60 alkyl;

(ii) $HO(R^{14})_2SiO(Si(R^{15})_2O)_xSi(R^{16})_2OH$, where $R^{14}$, $R^{15}$ and $R^{16}$ can be the same or different and are each independently selected from the group consisting of phenyl and C1-C60 alkyl; or

(iii) organo substituted silicon compounds of formula $R^{17}Si(R^{18})OSiR^{19}$ which are not polymeric where $R^{17}$, $R^{18}$ and $R^{19}$ can be the same or different and are each independently selected from the group consisting of phenyl and C1-C60 alkyl optionally with one or both of the terminal R groups also containing an hydroxyl group. Specific examples include dimethicone, dimethiconol behenate, $C_{30-45}$ alkyl methicone, stearoxytrimethylsilane, phenyl trimethicone and stearyl dimethicone.

(i) mixtures and blends of two or more of the foregoing.

The emollient or emollient mixture or blend thereof incorporated in compositions according to the present invention can, illustratively, be included in amounts of 0.5 - 50 %, preferably 1 - 25 %, more preferably 3 - 12 %, by weight, of the total weight of the composition.

Another category of optional ingredients useful in this invention is thickeners which may be added to increase the viscosity, improve aesthetics and/or improve the performance of the cosmetic compositions of the invention. Suitable thickeners include one or more members selected from the group consisting of:

(a) high melting point waxes (65-101 °C) such as beeswax, montan, ozokerite, ceresin, paraffin, hydrogenated castor oil, and C26-C50 linear alcohols;
(b) low melting point waxes (37-65 °C) such as fatty alcohols, fatty acids, fatty acid esters, fatty acid amides and particularly stearyl alcohol, cetyl alcohol, stearic acid and polydimethyl siloxanyl beeswax;
(c) silicone waxes such as methyl alkyl silicone waxes and ester silicone waxes, particularly stearoxytrimethylsilane, stearyldimethicone, dimethiconol behenate and C30-45 alkyl methicone;
(d) modified natural polymers such as those which are cellulose or guar based, particularly cellulose gum, hydroxyethylcellulose, methylcellulose, hydroxypropylmethylcellulose, hydroxypropyl guar;
(e) synthetic polymers such as alkylene/alkylene oxide polymers, particularly polyethylene, oxidized polyethylene, ethylene/acrylate copolymer, ethylene/vinyl acetate copolymer, PEG-n (where n is a number from 4-90,000) and PEG-m stearate (where m is a number from 2-175);
(f) inorganic smectite clays such as smectite clays and amorphous silicon dioxide particularly hectorite, sodium magnesium silicate, stearalkonium hectorite, Quatemium-18 hectorite, bentonite, magnesium aluminum silicate, Quatemium-18 bentonite, stearalkonium bentonite, hydrated silica, and silica;
(g) trihydroxystearin and tribehenin;
(h) mixtures and blends of any of the foregoing.

For thickeners of groups a, b, and c, the addition levels are in the range of 0.05-25 percent by weight (preferably 2-17 percent) based on the total weight of the composition. For thickeners of groups d, e, f and g, the addition levels are in the range of 0.01-10% by weight (preferably 0.1-5 percent) based on the weight of the total composition.

The amounts of surfactants, emollients and thickeners can be selected on the basis of the form desired and in combination with the amount of silicone gelling agent used.

Other various optional components include those described in US-A-5,019,375; US-A-4,937,069; and US-A-5,102,656. Examples of such additional ingredients include fragrances, insect repellents, coloring agents, opacificers, etc. in types and amounts conventionally used for such products.

The compositions of the invention may take various forms depending on the amount of silicone gel material and the amount of other ingredients used; some of the more preferable compositions are soft solids or gels. For example, a silicone gel may be made which comprises 1-30% by weight (based on the total weight of the silicone gel material) of at least one cross-linked organopolysiloxane material; and 70-99% by weight (based on the total weight of the silicone gel material) of the vehicle used (for example, cyclomethicone).

Compositions according to the present invention can be made by mixing the silicone gel material with surfactant(s), active ingredient(s) and optionally one or more of emollient(s), thickener(s) and fragrance. Mixing conditions and the use of heating will depend on what types of materials are being combined and; the melting points for those materials as are known to those skilled in the art. For example if soft solids, roll-ons or gels are being made, temperatures, in the range of room temperature or slightly higher (for example, 25-50 °C, particularly 23-30 °C) may be used. For stick products and soft solid/cream products made with higher melting point materials (for example, high temperature waxes) temperatures from 25-85 °C may be used. The mixture can be introduced into dispensing containers known to those skilled in the art including those for solids, gels, roll-ons, soft solids and creams. In one particular example, slotted dispensers may be used such as those known in the art, for example, those having a parallel row or rows of straight or curved slots or holes with a screw mechanism for forcing the composition through the top as the product is used.

Where the dispensing containers have a top surface with slots therein, the composition can be rubbed onto the skin from the top surface of the container (itself fed from a reservoir of product in the container) so as to deposit

an adequate amount of the cosmetic composition on to the skin. The cosmetic composition, for example. an antiperspirant and/or deodorant in the form of a soft solid, can be extruded from inside the dispensing container through the slots or holes onto the top of the surface of the dispensing container, and from there may be applied to the skin in the axillary regions to deposit sufficient amounts of antiperspirant and/or deodorant active material to reduce body malodor and/or reduce perspiration in axillary regions of the human body.

Various forms of the invention can be exemplified by the following formulations but should not be construed as limitations on the invention:

Antiperspirant Stick

1) 0.5-10% (preferably 2-8%) silicone gelling agent, for example, cyclopentasiloxane cetearyl dimethicone/vinyl crosspolymer.
2) 30-70% (preferably 40-50%) vehicle for the gelling agent, for example, volatile cyclic silicones.
3) 8-25% (preferably 20-22%) antiperspirant active, for example, a ZAG complex.
4) 0.5-10% (preferably 1-5%) surfactant selected from the group of nonionics (for example, Dimethicone copolyol, PEG 30 Castor oil, PEG 8 Dilaurate, PPG-1-PEG-9 Lauryl Glycol Ether, Dimethicone copolyol EO/PO polyether, Dimethicone copolyol laurate and mixtures thereof).
5) 1-15% (preferably 3-10%) emollients selected from the group consisting of fatty acid esters (for example, $C_{12-15}$ alkyl benzoate, diisopropyl adipate and neopentyl glycol diheptanoate), wherein a portion or all of the emollients may be combined to make the vehicle and any remainder portion of emollients may be added for an emollient effect.
6) thickeners such as one or more members selected from the group consisting of (a) 10-25% (preferably 14-22%) (for example, stearyl alcohol, isostearyl alcohol, cetyl alcohol, silicone waxes, hydrogenated castor oil and mixtures thereof) and (b) 0.1-5% (preferably 0.2-2.0%) of at least one of the members of the group consisting of silica, cellulose, polyethylene and mixtures thereof.
7) 0.05-5.0% (preferably 0.7-1.2%) fragrance.

Antiperspirant Gel/Soft solid/Cream

1) 0.5-10% (preferably 2-6%) silicone gelling agent, for example, cyclopentasiloxane cetearyl dimethicone/vinyl crosspolymer.
2) 40-80% (preferably 50-70%) vehicle for gelling agent, for example, volatile cyclic silicones.
3) 8-25% (preferably 20-22%) antiperspirant active, for example, a ZAG complex.
4) 1-8% (preferably 2-6%) surfactant selected from the group of nonionics, for example, Dimethicone copolyol, PEG 30 Castor oil, PEG 8 Dilaurate, PPG-1-PEG-9 Lauryl Glycol Ether, Dimethicone copolyol EO/PO polyether, Dimethicone copolyol laurate and mixtures thereof).
5) 1-15% (preferably 3-10%) emollients selected from the group consisting of fatty acid esters (for example, $C_{12-15}$ alkyl benzoate, diisopropyl adipate and neopentyl glycol diheptanoate), wherein a portion or all of the emollients may be combined to make the vehicle and any remainder portion of emollients may be added for an emollient effect.
6) thickeners such as one or more members selected from the group consisting of (a) 1-15% (preferably 2-8%) (for example, stearyl alcohol, isostearyl alcohol, cetyl alcohol, silicone waxes, hydrogenated castor oil and mixtures thereof) and (b) 0.1-5% (preferably 0.2-2.0%) of at least one of the members of the group consisting of silica, cellulose, polyethylene and mixtures thereof.
7) 0.05-5.0% (preferably 0.7-1.2%) fragrance.

Antiperspirant Roll on

1) 0.1-10% (preferably 0.5-3%) silicone gelling agent, for example, cyclopentasiloxane cetearyl dimethicone/vinyl crosspolymer.
2) 50-80% (preferably 60-70%) vehicle for gelling agent, for example, volatile cyclic silicones.
3) 8-25% (preferably 20-22%) antiperspirant active, for example, a ZAG complex.
4) 1-8% (preferably 2-6%) surfactant selected from the group of nonionics, for example, Dimethicone copolyol, PEG 30 Castor oil, PEG 8 Dilaurate, PPG-1-PEG-9 Lauryl Glycol Ether. Dimethicone copolyol EO/PO polyether,

Dimethicone copolyol laurate and mixtures thereof).
5) 1-15% (preferably 3-10%) emollients selected from the group consisting of fatty acid esters (for example, $C_{12-15}$ alkyl benzoate, diisopropyl adipate and neopentyl glycol diheptanoate), wherein a portion or all of the emollients may be combined to make the vehicle and any remainder portion of emollients may be added for an emollient effect.
6) thickeners such as one or more members selected from the group consisting of (a) 1-15% (preferably 2-8%) (for example, stearyl alcohol, isostearyl alcohol, cetyl alcohol, silicone waxes, hydrogenated castor oil and mixtures thereof) and (b) 0.1-5% (preferably 0.2-2.0%) of at least one of the members of the group consisting of silica, cellulose, polyethylene and mixtures thereof.
7) 0.05-5.0% (preferably 0.7-1.2%) fragrance.

**EXAMPLES**

The following Examples are offered as illustrative of the invention and are not to be construed as limitations thereon. In the Examples and elsewhere in the description of the invention, chemical symbols and terminology have their usual and customary meanings. In the Examples as elsewhere in this application values for n, m, etc. in formulas, molecular weights and degree of ethoxylation or propoxylation are averages. Temperatures are in °C unless otherwise indicated. The amounts of the components are in weight percents based on the standard described; if no other standard is described then the total weight of the composition is to be inferred. Various names of chemical components include those listed in the CTFA International Cosmetic Ingredient Dictionary (Cosmetics, Toiletry and Fragrance Association, Inc., 7th ed. 1997). Mixing techniques used to make the compositions are those conventionally used in the art including those described above.

Examples 1-81 - Preliminary Evaluation

Mixture 1 is made by combining 5.0% Cyclopentasiloxane Cetearyl Dimethicone/Vinyl Crosspolymer, 73.5% Cyclomethicone (70% pentamer + 30% hexamer), and 22.5% aluminum zirconium tetrachlorhydrex glycine complex (all amounts based on weight percents). A 2 gram samples of a test example is prepared by mixing 95% weight percent of Mixture 1 with 5% of a selected surfactant from the surfactants listed in Table I to make a series of Mixture 2's. A sample (0.1 gram) of each test example as Mixture 2 is blotted (2 cm diameter circular area) onto a swatch of black cloth (100% cotton fabric, 7.5 cm x 10 cm) which has been placed and taped on a black ceramic tile). The initial time is recorded. A Control sample is prepared without the surfactant and also applied to a fabric swatch in the same manner. Blot positions are marked for each one. The blots are allowed to dry for 20 minutes. Deionized water (1 drop) is applied to the top of each dried blot. The time needed for each water drop to be absorbed through the cloth is recorded (Absorption Time). This is apparent when the top surface of the drop is observed to be level with the flat surface of the cloth. The examples with absorption times less than 10 minutes and preferably less than 5 minutes were deemed to predict effectiveness in solubilizing film in the context of this invention . Correspondingly, those examples with absorption times > 45 minutes were deemed not to favor favorable solubilizing properties. The data obtained for evaluations of various surfactants as well as HLB values obtained through literature references are shown in Table 1 or experimentally determined using water solubility.

TABLE 1

| | | Absorption Time (minutes) | HLB |
|---|---|---|---|
| CONTROL (sample without surfactant) | | > 45 | N/A |
| Sorbitan & Ethoxylated Sorbitan Esters | | | |
| Ex.1. | Sorbitan Monolaurate (SPAN 20, ICI) | 22 | 8.6 |
| Ex.2. | Sorbitan Monopalmitate (SPAN 40, ICI) | 20 | 6.7 (Ref Ex) |
| Ex.3. | Sorbitan Monostearate (SPAN 60, ICI & Arlacel 60, ICI) | >45 | 2.1 (Ref Ex) |
| Ex.4. | Sorbitan Isostearate (CRILL 6, Croda) | 30 | 4.6 (Ref Ex) |
| Ex.5. | Sorbitan Monooleate (SPAN 80, ICI) | >45 | 4.3 (Ref Ex) |
| Ex.6. | Polysorbate 20 (TWEEN 20, ICI) | 5 | 16.7 (Ref. Ex) |
| Ex.7. | Polysorbate 40 (TWEEN 40, ICI) | 1 | 15.6 |
| Ex.8. | Polysorbate 60 (TWEEN 60. ICI) | 1 | 14.9 |
| Ex.9. | Polysorbate 80 (TWEEN 80. ICI) | 5 | 15.0 |
| Ex.10. | Polysorbate 120 (CRILLET 6. Croda) | 3 | 14.9 |

TABLE 1 (continued)

| | | Absorption Time (minutes) | HLB |
|---|---|---|---|
| Sorbitan & Ethoxylated Sorbitan Esters | | | |
| Ex. 11. | PEG-20 Sorbitan Beeswax (G-1726, ICI) | 3 | |
| Ethoxylates | | | |
| Ex.12. | Ceteth 20 (Cetomacrogol 1000 BP, Croda) | 3 | 15.6 |
| Ex.13. | PEG 30 Castor oil (INCROCAS 30, Croda) | 3 | 13.0 |
| Ex.14. | PEG 40 Hydrogenated Castor oil (CREMAPHOR RH-40, BASF) | 3 | 14-16 |
| Ex.15. | PEG 60 Hydrogenated Castor oil (CREMAPHOR RH-60, BASF) | 3 | 14-16 |
| Ex.16. | Ceteareth 12 (EMULGEN B-1, Henkel) | 3 | |
| Ex.17. | Ceteareth 20 (INCROPOL CS-20, Croda) | 1 | 15.5 |
| Ex.18. | Ceteareth 20 (EMULGEN B-2, Henkel) | 1 | 15.5 |
| Ex.19. | PPG 5 Ceteth 20 (PROCETYL AWS, Croda) | 4 | 16.0 |
| Ex.20. | PPG 3 Buteth 5 (UCON50-HB-100 Union Carbide) | 3 | 13+ |
| Ex.21. | Steareth 2 (VOLPO S-2, Croda) | 5 | 4.9 (Ref Ex) |
| Ex.22. | Steareth 2 (BRIJ 72, ICI) | 5 | 4-6 (Ref Ex) |
| Ex.23. | Steareth-10 (VOLPO S-10, Croda) | 3 | 12.4 |
| Ex.24. | Steareth-20 (VOLPO S-20, Croda) | 1 | 15.5 |
| Ex.25. | Steareth-21 (BRIJ 721S, ICI) | 8 | 14-16 |
| Ex.26. | Steareth-100 (BRIJ 700, ICI) | 15 | 18.8 (Ref Ex) |
| Ex.27. | Oleth-5 (VOLPO 5, Croda) | 0.5 | 8.8 |
| Ex. 28. | Oleth-9 (Phenoxolol-9. Phoenix Chemical Company) | 0.5 | 10-13 |
| Ex.29. | Oleth-10 (VOLPO 10. Croda) | 1 | 12.4 |
| Ex.30. | Oleth-20 (RHODASURF ON-870, Rhone Poulenc) | 8 | 15.4 |
| Ex.31. | Oleth-20 (RHODASURF ON-877, Rhone Poulenc) | 3 | 15.4 |
| Ethoxylated Adducts | | | |
| Ex.32. | PEG 25 PG Stearate (G-2162, ICI) | 4 | 11.0 |
| Ex.33. | Glyceryl Stearate and PEG 100 Stearate (ARLACEL 165, ICI) | 30 | 11.0 |
| Alcohol Esters | | | |
| Ex.34. | Myristyl Myristate (ALKAMULS MM/M, Rhone Poulenc) | >45 | |
| Glycol Esters | | | |
| Ex.35. | Glyceryl Monostearate (CUTINA GMS, Henkel) | >45 | |
| PEG Esters | | | |
| Ex.36. | PEG 8 Oleate (ALKAMULS 400-MO, Rhone Poulenc) | 1 | 11.0 |
| Ex.37. | PEG 12 Dilaurate (JEEMATE 600-DL, Jeen) | I | |
| Ex.38. | PEG 8 Dilaurate (JEEMATE 400-DL, Jeen) | 1 | 10-13 |
| Ex.39. | PEG 8 Laurate (JEEMATE 400-ML, Jeen) | 1 | 13+ |
| Ex.40. | PEG 8 Distearate (PEG-400 Distearate, Stepan) | 6 | 10-13 |
| Ex.41. | PEG 40 Stearate (MYRJ 52, ICI) | 5 | 15-17 (Ref. Ex.) |
| Ex.42. | PEG 80 Diisostearate (PEG 4000 Diisostearate, Scher) | 35 | |

TABLE 1 (continued)

| | | Absorption Time (minutes) | HLB |
|---|---|---|---|
| Propoxylates | | | |
| Ex.43. | PPG 10 Butanediol (Probutyl DB-10. Croda) | 5 | 10-13 |
| Ex.44. | PPG 10 Cetyl ether (Procetyl 50. Croda) | >45 | 1-4 (Ref Ex) |
| Ex.45. | PPG 11 Stearyl ether (WITCONOL APS, Witco) | 10 | 1-4 (Ref Ex) |
| Ex.46. | PPG 3 Myristyl ether ( Promyristyl PM-3) | 10 | 1-4 (Ref Ex) |
| Ex.47. | PPG 50 Oleyl ether (PROVOL 50, Croda) | 30 | 1-4 (Ref Ex) |
| Ex.48. | PPG-1-PEG-9 Lauryl Glycol Ether (EUMULGIN L, Henkel) | 1 | 13+ |
| Ex.49. | PPG 2 Myristyl ether propionate (CRODAMOL PMP, Croda) | >45 | 1-4 (Ref Ex) |
| Alkylphenol Aromatic Ethoxylates | | | |
| Ex.50. | Octylphenol Ethoxylate (IGEPAL CA-877, Rhone Poulenc) | 45 | 17.4 (Ref. Ex.) |
| Ex.51. | Octylphenol Ethoxylate (IGEPAL CA897, Rhone Poulenc) | >45 | 18.0 (Ref. Ex.) |
| Ex.52. | Dinonylphenol Ethoxylate (IGEPAL DM-530, Rhone Poulenc) | 1 | 9.4 |
| Block Copolymers | | | |
| Ex.53. | Poloxamer 182 (ANTAROX L-62, Rhone Poulenc) | 6 | 7.0 (Ref Ex) |
| Ex.54. | Poloxamer 234 (ANTAROX P-84, Rhone Poulenc) | 3 | 14.0 |
| Ex.55. | Meroxipol 174 (ANTAROX 17-R-4, Rhone Poulenc) | >45 | 4-7 (Ref Ex) |
| Silicone Polyethers | | | |
| Ex.56. | Polydimethicone Copolyol (SILSOFT 477. Witco Organosilicones) | 7 | 1-4 (Ref Ex) |
| Ex.57. | Dimethicone Copolyol, (SILWET L-7622. Witco Oreanosilicones) | 25 | 5-8 (Ref Ex) |
| Ex.58. | Dimethicone Copolyol, (SILWET L-7087. Witco Organosilicones) | 1 | 9-12 |
| Ex.59. | Dimethicone Copolyol, (SILWET L-7200, Witco Organosilicones) | 2 | 13-17 (Ref. Ex) |
| Ex.60. | Dimethicone Copolyol, (SILWET L-7657, Witco Organosilicones) | 1 | 13-17 (Ref. Ex.) |
| Ex.61. | Eugeno Dimethicone Copolyol (SILSOFT Shine, Witco Organosilicones) | I | 10-13 |
| Ex.62. | Polyglyceryl-4 Isostearate & Cetyl Dimethicone Copolyol & Hexyl Laurate (ABIL WE 09, Goldschmidt) | 15 | 1-4 (Ref Ex) |
| Ex.63. | Dimethiconol Stearate (SILWAX S, Lambent Technologies) | >45 | |
| Ex.64. | Dimethiconol Castorate (SILWAX C, Lambent Technologies) | >45 | |
| Ex.65. | Dimethicone Copolyol Isostearate (SILWAX WD-IS, Lambent Technologies) | 1 | |
| Ex.66. | Dimethicone Copolyol Laurate (SILWAX WS-L, Lambent Technologies) | 1 | 9.4 |

TABLE 1 (continued)

| | | Absorption Time (minutes) | HLB |
|---|---|---|---|
| Silicone Polyethers | | | |
| Ex.67 | Dimethicone Copolyol (ABIL B 8830, Goldschmidt) | 7 | 3-6 (Ref Ex) |
| Ex.68 | Dimethicone Copolyol (ABIL B 8852, Goldschmidt) | 1 | 6-8 (Ref Ex) |
| Ex.69 | Dimethicone Copolyol (ABIL B 8863, Goldschmidt) | 7 | 13+ |
| Ex.70 | Dimethicone Copolyol (SILWET L-7001, Witco) | 2 | 9-12 |
| Ex.71 | Dimethicone Copolyol (SILWET L-7002, Witco) | 2 | 9-12 |
| Ex.72 | Dimethicone Copolyol (SILWET L-7230, Witco) | 3 | 9-12 |
| Ex.73 | Dimethicone Copolyol (SILWET L-7280, Witco) | 10 sec | 8 |
| Ex.74 | Dimethicone Copolyol (SILWET L-7500, Witco) | 10 | 5 (Ref Ex) |
| Ex.75 | Dimethicone Copolyol (SILWET L-7600, Witco) | 1.5 | 13-17 (Ref. Ex) |
| Ex.76 | Dimethicone Copolyol (SILWET L-7602, Witco) | 5 sec | 8 |
| Ex.77 | Dimethicone Copolyol (SILWET L-7604, Witco) | 1 | 13-17 (Ref. Ex) |
| Ex.78 | Dimethicone Copolyol (SIL.WET L-7607, Witco) | 1 | 13-17 (Ref. Ex) |
| Ex.79 | Dimethicone Copolyol (SILWET L-7608, Witco) | 10 sec | 8 |
| Ex.80. | Dimethicone Copolyol (DOW 190, Dow Coming) | 20 sec | 10-13 |
| Ex.81 | Dimethicone Copolyol (DOW 193, Dow Coming) | 20 sec | 13+ |

Examples 82-97 Second Level Evaluation: Nature of the Film

After the evaluation described in Examples 1-81 was completed, a second level test was used to demonstrate the effectiveness of selected surfactants to form a solubilizable film by evaluating the nature of the film in selected compositions. A 1 kilogram batch was prepared for selected sample formulations using the amounts of materials listed in Tables 2-5 below. Silicone gel material was added to a beaker. The surfactants (such as PEG-8 distearate, dimethicone copolyol) were added. Next the active cosmetic material was added. The ingredients were added in the order listed above and mixed with homogenization. A portion of each sample (as described in the compositions in Tables 2 - 5) (1 gram) is applied evenly on the bottom of a petri dish. The coated dish is placed in an oven (37 °C) for one hour. The dish is removed from the oven and distilled water (10 gm) is then added to the dish . The sample is shaken gently for two hours. Water is decanted from the dish and the dish is then allowed to air dry. Hydrophobicity/hydrophilicity on dried sample was obtained by placing a drop of water and visually examining for droplet shape (also characterized in having a contact angle greater or less than 90 degrees). This position may be evaluated by measuring the contact angle between a liquid (water), a substrate (film on petri dish, fiber surface, etc.) and air. For hydrophobic films, the contact angle is greater than 90 degrees because the water does not wet the film and thus beads up. For hydrophilic films, the contact angle is less than 90 degrees because the water wets the film and increases contact. The data for this evaluation is found in Tables 2- 5; those samples noted as being hydrophilic are preferred.

Examples - 82-97 Third Level Evaluation: Release of Active

The types of samples described above under Examples 82-97 - Second Level Evaluation were also subjected

to a further test. Since release of the active from the matrix of an antiperspirant is necessary for clinical efficacy of antiperspirant products, an evaluation of the quantity of aluminum and zirconium released from the antiperspirant matrix in in vitro conditions may be performed. For sample preparation cotton pads (10 cm x 10 cm), made of Webril® cotton pads (Kleantest) are cut and an application zone, in the form of the disk (7 cm diameter) is marked in the center of the cotton pad. A thin layer of silicone elastomer composition (0.9 gram) is applied to the application zone. The silicone elastomer composition is dried to about 10% of its original weight. A sample (0.75 grams) of antiperspirant product is applied on a Webril® pad and the pad is folded into a 3.3 cm square. The sample holder is then placed into a dissolution vessel of the dissolution apparatus. A U.S.P. dissolution apparatus (VK7000™, Vankel Industries, Cary, NC) is used to study the dissolution of the aluminum/zirconium complexes from the antiperspirant product. The dissolution medium (200 ml of deionized, degassed water) is placed in 200 ml dissolution vessels. Stirring of the medium is done with 200 rpm rotation of stainless steel paddles (type 316 1/4" ( 0.64 cm) diameter shaft). The vessels are submerged in a temperature controlled (37.5°C) water bath. Sampling of the medium (5 ml) is obtained by a dissolution sampling station (VK 8000™, Vankel Industries). The sample aspiration is provided by a bidirectional peristaltic pump. Following sampling, the sampled volume is replaced with fresh medium. Sampling periods are each set at 120 minutes. Samples are collected in disposable culture tubes (Kimax® 51, borosilicate glass 16x100), and 5ml of a 10% (weight/weight) nitric acid solution in water are added to provide complete solubility of the Al and Zr species. The samples are then analyzed by Inductively Coupled Plasma (ICP), and the concentration of aluminum and zirconium is calculated down to a sensitivity of 0-100 ppm. Standards of aluminum and zirconium at concentrations of 50 ppm and 100 ppm may be included in the calibration of the equipment. The results are expressed as a function of the % Al or % Zr released based on the total amount of aluminum and zirconium contained in the products. The concentration measured by ICP is converted into a measurement of total aluminum amount released measured by ICP:

$$Q_{ICP} = C_{ICP} \times 200 \times 2$$

where $Q_{ICP}$ = the quantity of aluminum detected by ICP and $C_{ICP}$ = the concentration of aluminum detected by ICP. The % released is a function of the amount of aluminum released and the total aluminum present in the sample as per the formula:

$$\%_{Release} = 100 \times Q_{ICP}/Q_0$$

where $Q_0$ is the original amount of the aluminum in the sample (in grams). $Q_0$ can be calculated using the following formula:

$$Q_0 = \text{\% aluminum in salt (COA)} \times \text{(\% salt in formula)} \times \text{(sample /10000)}$$

COA = the value from the certificate of analysis of the antiperspirant active. Measurements were taken 120 minutes after application. The data is found in Tables 2- 5 under Release %. The samples showing the higher amounts of release compared to the control are preferred.

**Table 2**

| Ingredients | Control #1 | Ex. 82 | Ex. 83 | Ex. 84 | Ex. 85 |
|---|---|---|---|---|---|
| Cyclomethicone / Elastomer Blend | 65.05 | 65.05 | 65.05 | 65.05 | 65.05 |
| Aluminum-Zirconium Tetrachlorohydrex gly | 22.5 | 22.5 | 22.5 | 22.5 | 22.5 |
| Ex. 80 Surfactant | | 2 | | | |
| Ex. 81 Surfactant | | | 2 | | |
| Ex. 66 Surfactant | | | | 2 | |
| Ex. 59 Surfactant | | | | | 2 |
| q.s. Cyclomethicone (SF 1202) | 12.45 | 10.45 | 10.45 | 10.45 | 10.45 |
| Release % | 72 | 71 | 69 | 83 | 79 |
| Nature of the Film | Hydrophobic | Hydrophilic | Hydrophilic | Hydrophilic | Hydrophilic |

**Table 3**

| Ingredients | Control #1 | Ex. 86 | Ex. 87 | Ex. 88 | Ex. 89 |
|---|---|---|---|---|---|
| Cyclomethicone / Elastomer Blend | 65.05 | 65.05 | 65.05 | 65.05 | 65.05 |
| Aluminum-Zirconium Tetrachlorohydrex gly | 22.5 | 22.5 | 22.5 | 22.5 | 22.5 |
| Ex. 48 Surfactant | | 2 | | | |
| Ex. 61 Surfactant | | | 2 | | |
| Ex. 19 Surfactant | | | | 2 | |
| Ex. 13 Surfactant | | | | | 2 |
| q.s. Cyclomethicone (SF 1202) | 12.45 | 10.45 | 10.45 | 10.45 | 10.45 |
| Release % | 72 | 81 | 67 | 67 | 74 |
| Nature of the Film | Hydrophobic | Hydrophilic | Hydrophilic | Hydrophilic | Hydrophilic |

Table 4

| Ingredients | Control #1 | Ex. 90 | Ex. 91 | Ex. 92 | Ex. 93 |
|---|---|---|---|---|---|
| Cyclomethicone / Elastomer Blend | 65.05 | 65.05 | 65.05 | 65.05 | 65.05 |
| Aluminum-Zirconium Tetrachlorohydrex gly | 22.5 | 22.5 | 22.5 | 22.5 | 22.5 |
| Ex. 32 Surfactant | | 2 | | | |
| Ex. 37 Surfactant | | | 2 | | |
| Ex. 31 Surfactant | | | | 2 | |
| Ex. 36 Surfactant | | | | | 2 |
| q.s. Cyclomethicone (SF 1202) | 12.45 | 10.45 | 10.45 | 10.45 | 10.45 |
| Release % | 72 | 66 | 63 | 56 | 66 |
| Nature of the Film | Hydrophobic | Hydrophilic | Hydrophilic | Hydrophobic | Hydrophilic |

Table 5

| Ingredients | Control #1 | Ex. 94 | Ex. 95* | Ex. 96 | Ex. 97 |
|---|---|---|---|---|---|
| Cyclomethicone / Elastomer Blend | 65.05 | 65.05 | 65.05 | 65.05 | 65.05 |
| Aluminum-Zirconium Tetrachlorohydrex gly | 22.5 | 22.5 | 22.5 | 22.5 | 22.5 |
| Ex. 24 Surfactant | | 2 | | | |
| Ex. 6 Surfactant | | | 2 | | |
| Ex. 7 Surfactant | | | | 2 | |
| Ex. 60 Surfactant | | | | | 2 |
| q.s. Cyclomethicone (SF 1202) | 12.45 | 10.45 | 10.45 | 10.45 | 10.45 |
| Release % | 72 | 54 | 59 | 59 | 66 |
| Nature of the Film | Hydrophobic | Hydrophobic | Hydrophilic | Hydrophilic | Hydrophilic |

* reference example (with surfactant of HLB>16)

<u>Examples AA - DD</u> - Antiperspirant/Deodorant Cream: Comparison of Invention

Compositions may be made using the ingredients listed in Table 6.

TABLE 6

| | | | | Ref. Ex |
|---|---|---|---|---|
| **Ingredient** | **Example AA** | **Example BB** | **Example CC** | **Example DD** |
| Cyclopentasiloxane (and) Cetearyl Dimethicone/ Vinyl Dimethicone Crosspolymer | 64.25 | 65.05% | 65.25 | 63.05 |
| Aluminum zirconium tetrachlorohydrex gly | 22.50 | 22.50 | 25.00 | 25.00 |
| Neopentyl glycol diheptanoate | 0.00 | 5.00 | 0.00 | 0.00 |
| C12-15 alkyl benzoate | 3.00 | 3.00 | 8.25 | 0.00 |
| PEG-8 distearate | | 2.00 | | |
| Diisopropyl adipate | 5.00 | 0.00 | 0.00 | 0.00 |
| Phenyl trimethicone | 0.00 | 0.00 | 0.00 | 8.25 |
| Dimethicone copolyol laurate | 2.00 | 0.00 | 0.00 | 2.00 |
| Dimethicone copolyol | 0.00 | 1.00 | 1.00 | 1.00 |
| Stearyl dimethicone | 0.75 | 0.75 | 0.00 | 0.00 |
| Silica | 0.00 | 0.20 | 0.00 | 0.20 |
| Fragrance | 0.50 | 0.50 | 0.50 | 0.50 |
| Total | 100.00 | 100.00 | 100.00 | 100.00 |

For evaluating aesthetic properties, a composition made with the composition of Example BB was compared on the basis of aesthetics with Example 4 from WO 97/44010. Sample BB was a soft solid having the composition described in Table 6. A 1 kilogram batch was prepared for selected sample formulations using the amounts of materials listed in Table 6. Silicone gel material was added to a beaker. The surfactants (such as PEG-8 distearate, dimethicone copolyol) and emollients (such as C12-15 alkyl benzoate and neopentyl glycol diheptanoate) were added. Next the active cosmetic material was added. Next the thickeners (for example, silica or stearyl dimethicone) were added. Finally the fragrance was added. The ingredients were added in the order listed above and mixed with homogenization. Sample XX was made with 70% Gransil GCM™, 20% Al-Zr tetrachlorohydrex gly and 10% Dimethicone. A group of 20 in-house panelists was used. For each individual, a portion of each of Sample BB and Sample XX (about 0.2 grams of each) was applied at the back of each arm. The area of the forearm is marked. Both samples were applied by the panelists scooping each sample from a dish with the forefinger and rubbing the sample with upward/downward strokes ten times on the marked area. Evaluations were made and the results are shown in Table 7. The numbers indicate the number of panelists responding in the indicated category. The data shows the superiority of a composition of the invention.

TABLE 7

| Attribute | Sample BB | Sample XX |
|---|---|---|
| Less Wet | 7 | 12 |
| Less Oily | 6 | 14 |
| More Drag - During Application | 5 | 14 |
| More Drag- Immediately After Application | 5 | 14 |
| More Drag - After 5 Minutes | 3 | 16 |
| More Tack | 7 | 13 |

TABLE 7 (continued)

| Attribute | Sample BB | Sample XX |
|---|---|---|
| Less Whitening | 16 | 4 |

Examples EE-GG

The conventional mixing methods described above were used to make products with the ingredients shown in Table 8.

TABLE 8

| **Ingredient** | **Example EE** | **Example FF** | **Example GG** |
|---|---|---|---|
| Cyclopentasiloxane (and) Cetearyl Dimethicone/Vinyl Dimethicone Crosspolymer | 63.05 | 62.50 | --- |
| Cyclopentasiloxane (and) Cetearyl Dimethicone Crosspolymer | --- | --- | 60.8 |
| Aluminum Zirconium Tetrachlorohydrex Glycine Complex | 22.50 | 22.50 | 22.50 |
| C12-15 Alkyl Benzoate | 4.00 | 5.00 | 7.5 |
| C12-15 Alkyl Octanoate | --- | 5.00 | 7.5 |
| Neopentyl Glycol Diheptanoate | 5.00 | --- | --- |
| PPG-1-PEG-9 Lauryl Glycol Ether | 2.00 | --- | --- |
| Poloxamer 105 Benzoate | --- | 2.00 | --- |
| PEG-8 Distearate | 2.00 | --- | --- |
| Stearyl Dimethicone | 0.75 | --- | --- |
| Polyethylene | --- | 2.95 | 1.50 |
| Silica | 0.20 | 0.05 | 0.20 |
| Fragrance | 0.50 | --- | --- |
| Total | 100.00 | 100.00 | 100.00 |

**Claims**

**1.** A cosmetic antiperspirant and/or deodorant composition made by combining:

(a) at least one antiperspirant active material;
(b) a silicone gel material which is made by combining:

(i) a crosslinked organopolysiloxane material as a gelling agent wherein the organopolysiloxane is made from at least one silicone hydride cross-linking agent and at least one member selected from the group consisting of (A) a vinyl polysiloxane and (B) an alpha, omega diene; and
(ii) a liquid base vehicle for the gelling agent wherein the vehicle is made by combining at least one member of the group consisting of volatile silicone materials and non-volatile silicone materials, and optionally wherein at least one liquid emollient which is soluble or miscible in the selected silicone material(s) is added to form the vehicle;

(c) at least one nonionic surfactant having an HLB value of 8-16;
and
(d) at least one emollient material selected from the group consisting of isopropyl myristate, isopropyl palmitate, isopropyl stearate, isopropyl isostearate, butyl stearate, octyl stearate, hexyl laurate, cetyl stearate, diisopropyl adipate, isodecyl oleate, diisopropyl sebacate, isostearyl lactate, $C_{12-15}$ alkyl benzoate, myreth-3 myristate,

dioctyl malate, neopentyl glycol diheptanoate, neopentyl glycol dioctanoate, dipropylene glycol dibenzoate, $C_{12-15}$ alcohols lactate, isohexyl decanoate, isohexyl caprate, diethylene glycol dioctanoate, octyl isononanoate, isodecyl octanoate, diethylene glycol diisononanoate, isononyl isononanoate, isostearyl isostearate, behenyl behenate, $C_{12-15}$ alkyl fumarate, laureth-2-benzoate, propylene glycol isoceteth-3 acetate, propylene glycol ceteth-3 acetate, octyldodecyl myristate, cetyl recinoleate, and myristyl myristate.

**2.** A cosmetic composition according to claim 1 wherein the nonionic surfactant has an HLB value of 8-12.

**3.** A cosmetic composition according to claim 1 wherein the antiperspirant active is selected from the group consisting of aluminum chlorohydrate, aluminum chloride, aluminum sesquichlorohydrate; zirconyl hydroxychloride; aluminum-zirconium glycine complex itself selected from the group consisting of for example, aluminum zirconium trichlorohydrex gly, aluminum zirconium pentachlorohydrex gly, aluminum zirconium tetrachlorohydrex gly and aluminum zirconium octochlorohydrex gly; aluminum chlorohydrex PG; aluminum chlorohydrex PEG; aluminum dichlorohydrex PG; aluminum dichlorohydrex PEG; aluminum nitratohydrate and its combination with zirconyl hydroxychlorides and nitrides; and aluminum-stannous chlorohydrates.

**4.** A cosmetic composition according to claim 3 wherein the antiperspirant active is added to the cosmetic composition in an amount in the range of 0.1-30 percent by weight based on the total weight of the composition.

**5.** A cosmetic composition according to any of the preceding claims, wherein the antiperspirant active material is combined with an antimicrobial agent selected from the group consisting of bacteriostatic quaternary ammonium compounds, cetyltrimethylammonium bromide, cetyl pyridinium chloride, 2,4,4'-trichloro-2'-hydroxydiphenylether, N-(4-chlorophenyl)-N'-(3,4-dichlorophenyl)urea, silver halides, octoxyglycerin, and zinc salts.

**6.** A cosmetic composition according to any one of the preceding claims, wherein the crosslinked organopolysiloxane material is made from at least one crosslinking agent and at least one vinyl polysiloxane.

**7.** A cosmetic composition according to claim 1 wherein the crosslinked organopolysiloxane material is made from at least one crosslinking agent and at least one alpha, omega dierie.

**8.** A cosmetic composition according to claim 6, wherein the vinyl polysiloxane is selected from the group consisting of:

(a) vinyl terminated polysiloxanes of Formula I:

$$CH_2{=}CH\text{-}Si(CH_3)_2 - O - (-Si(OH_3)_2 -O - )_n - Si(CH_3)_2 - CH{=}CH_2 \qquad \text{Formula I}$$

and
(b) vinyl functional copolymers of Formula II:

$$(CH_3)_3\ Si\text{-}\ O - (Si(CH_3)_2\text{-}O - )m - (Si(CH_3)(CH{=}CH_2))_n\text{-}Si(CH_3)_3 \qquad \text{Formula II}$$

where n = a number from 1-100 and m = a number from 1-100.

**9.** A cosmetic composition according to claim 8 wherein n = a number from 10-50.

**10.** A cosmetic composition according to claim 8 wherein m = a number from 10-50.

**11.** A cosmetic composition according to claim 8 wherein the vinyl polysiloxane comprises at least one member selected from the group consisting of:

(a) polydimethylsiloxane, which is monovinyl terminated;
(b) vinylmethyl, dimethylsiloxane copolymer which is trimethylsiloxy terminated;
(c) vinylmethyl, dimethylsiloxane copolymer which is vinyl dimethyl terminated;
(d) divinylmethyl terminated polydimethyl siloxanes;
(e) vinyl Q-resin

(f) vinylphenylmethyl terminated dimethyl siloxanes;
(g) cyclicvinylmethyl dimethyl siloxanes;
(h) T-structure polydimethyl siloxanes with vinyl at branchpoint;
(i)T-structure polydimethyl siloxane with vinyl at branch terminus;
(j) diphenyl dimethyl copolymer which is vinyl terminated;
(k) vinyl terminated polymethyl siloxanes;
(l) vinyl terminated trifluoropropyl methyl siloxane - dimethylsiloxane copolymer;
(m) vinyl terminated diethyl siloxane copolymer;
(n) vinyl methyl siloxane - dimethyl siloxane copolymer which is trimethylsiloxy terminated;
(o) vinyl gums; and
(p) vinyl methyl siloxane homopolymers.

**12.** A cosmetic composition according to claim 7 wherein the alpha, omega diene is selected from the group consisting of Formula:

$$CH_2=CH(CH_2)_xCH=CH_2,$$

where x is a number in the range of 1-20.

**13.** A cosmetic composition according to claim 7 wherein the alpha, omega diene is selected from the group consisting of 1,4-pentadiene; 1,5-hexadiene; 1,6-heptadiene; 1,7-octadiene; 1,8-nonadiene, 1,11-dodecadiene; 1,13-tetradecadiene; and 1,19-eicosadiene.

**14.** A cosmetic composition according to claim 1 wherein the crosslinking agent is at least one member selected from the group consisting of hydride terminated polydimethylsiloxanes of Formula III:

$$H\text{-}Si(CH_3)_2\text{-} O \text{-} (Si(CH_3)_2\text{-}O\text{-})_p\text{-} Si(CH_3)_2\text{-}H \qquad \text{Formula III}$$

where p= a number from 1-50.

**15.** A cosmetic composition according to claim 14 wherein p= a number from 5-20.

**16.** A cosmetic composition according to claim 1 wherein the cross-linking agent is at least one member selected from the group consisting of

(a) methylhydro-dimethylsiloxane copolymer with 20-60% methyl hydrogen;
(b) methylhydrosiloxane - dimethylsiloxane copolymer;
(c) polymethylhydrosiloxanes;
(d) polyethylhydrosilane;
(e) polyphenyl - (dimethylhydrosiloxy)siloxane which is hydride terminated;
(f) methylhydrosiloxane - phenylmethylsiloxane copolymer which is hydride terminated; and
(g) methylhydrosiloxane - octylmethylsiloxane copolymer.

**17.** A cosmetic composition according to claim 1 wherein the volatile and non-volatile silicone materials are selected from the group consisting of cyclic and linear volatile and non-volatile silicones.

**18.** A cosmetic composition according to claim 17 wherein the volatile silicones are one or more members selected from the group consisting of cyclic polydimethylsiloxanes represented by Formula IV

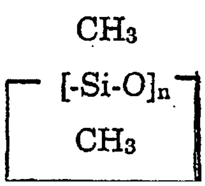

Formula IV

where n is an integer with a value of 3-7.

**19.** A cosmetic composition according to claim 17 wherein the nonvolatile and volatile linear silicones are one or more members selected from the group consisting of linear polydimethylsiloxanes represented by Formula V:

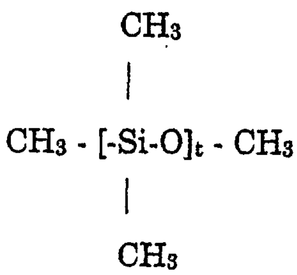

Formula V

wherein t is selected to that molecular weight is in the range of 800-260,000 and viscosity is in the range of 0.05-6000 $cm^2/s$ (5-600,000 centistokes).

**20.** A cosmetic composition according to claim 1 wherein the nonionic surfactant is at least one member selected from the group consisting of:

(a) sorbitan esters and ethoxylated sorbitan esters;
(b) ethoxylates;
(c) ethoxylated adducts;
(e) propoxylates;
(f) ethoxylated modified triglycerides;
(g) alkylphenol aromatic ethoxylates;
(h) block copolymers which are alkoxylated glycols having ethoxylated and propoxylated segments;
(i) silicone polyethers.

**21.** A cosmetic composition according to claim 20 wherein

(a) the sorbitan esters and ethoxylated sorbitan esters are selected from the group consisting of PEG-20 sorbitan isostearate, sorbitan monolaurate, polysorbate-40, polysorbate-60, polysorbate-80, and polysorbate-120;
(b) the ethoxylates are selected from the group consisting of Ceteth-20, PEG-30 castor oil, PEG-40 hydrogenated castor oil, PEG-60 hydrogenated castor oil, Laureth-7, Isolaureth-6, Steareth-10, Steareth-20, Steareth-21, Ceteareth-12, Ceteareth-20, Oleth-5, Oleth-9, Oleth-10, Oleth-20, and PPG-3-Buteth-5,
(c) the ethoxylated adducts are selected from the group consisting of PEG-25 stearate,glyceryl stearate and PEG-100 stearate;
(e) the propoxylates are selected from the group consisting of PPG-10 butanediol, PPG-1-PEG-9 Lauryl Glycol Ether, PPG-3-deceth-3, and PPG-5-ceteth-20;
(f) the ethoxylated modified triglycerides are selected from the group consisting of PEG-20 corn glycerides, and PEG-12 palm kernel glycerides;
(g) the alkylphenol aromatic ethoxylates are selected from the group consisting of dinonylphenol ethoxylated with 9 moles of ethylene oxide, octylphenol ethoxylated with 20 moles of ethylene oxide, and octylphenol ethoxylated with 40 moles of ethylene oxide;
(h) the block copolymers are selected from the group consisting of alkoxylated glycols having ethoxylated and propoxylated segments, Poloxamer 182 and Poloxamer 234;
(i) the silicone polyethers are selected from the group consisting of (1) dimethicone copolyols; (2) dimethicone copolyol laurates; (3) dimethicone copolyol isostearates; (4) polydimethicone copolyols; and (5) eugenodimethicone copolyols.

**22.** A cosmetic composition according to claim 1 made by forming the silicone gel material with 1-30% by weight of at least one cross-linked organopolysiloxane material and 70-99% by weight of the vehicle, based on the total weight of the silicone gel material.

**23.** A cosmetic composition according to claim 1 made with 0.1-20% surfactant based on the total weight of the composition.

**24.** A cosmetic composition according to claim 1 made by combining 10-80% silicone gel material; 0.1-30% antiperspirant active; 0.1-20% surfactant.

**25.** A cosmetic composition according to claim 24 wherein the vehicle is made by combining at least one silicone material with 0.6-50% of at least one emollient material.

**26.** A cosmetic composition according to claim 25, wherein the composition contains at least one thickener.

**Patentansprüche**

**1.** Kosmetische Antiperspirant- und/oder Deodorantzusammensetzung, die hergestellt wurde, indem kombiniert wurden:

(a) mindestens ein als Antiperspirant wirkendes Material,

(b) ein Silikongelmaterial, das hergestellt worden ist, indem kombiniert wurden:

(i) ein vernetztes Organopolysiloxanmaterial als ein Gelierungsmittel, wobei das Organopolysiloxan aus mindestens einem Silikonhydrid-Vernetzungsmittel und mindestens einem Mitglied aus der Gruppe bestehend aus (a) einem Vinylpolysiloxan und (b) einem alpha,omega-Dien hergestellt worden ist, und

(ii) ein flüssiger Basisträger für das Geliermittel, wobei der Träger hergestellt worden ist, indem mindestens ein Mitglied aus der Gruppe bestehend aus flüchtigen Silikonmaterialien und nichtflüchtigen Silikonmaterialien miteinander kombiniert werden, und wobei gegebenenfalls mindestens ein flüssiges Aufweichmittel, das in dem ausgewählten Silikonmaterial (den ausgewählten Silikonmaterialien) löslich oder damit mischbar ist, zugesetzt wird, um den Träger zu bilden,

(c) mindestens ein nichtionisches Tensid mit einem HLB-Wert von 8 - 16, und

(d) mindestens ein Aufweichmaterial ausgewählt aus der Gruppe bestehend aus Isopropylmyristat, Isopropylpalmitat, Isopropylstearat, Isopropylisostearat, Butylstearat, Octylstearat, Hexyllaurat, Cetylstearat, Diisopropyladipat, Isodecyloleat, Diisopropylsebacat, Isostearyllactat, $C_{12-15}$-Alkylbenzoat, Myreth-3-myristat, Dioctylmaleat, Neopentylglykoldiheptanoat, Neopentylglykoldioctanoat, Dipropylenglykoldibenzoat, $C_{12-15}$-Alkohollactat, Isohexyldekanoat, Isohexylcaprat, Diethylenglykoldioctanoat, Octylisononanoat, Isodecyloctanoat, Diethylenglykoldiisononanoat, Isononylisononanoat, Isostearylisostearat, Behenylbehenat, $C_{12-15}$-Alkylfumarat, Laureth-2-benzoat, Propylenglykolisoceteth-3-acetat, Propylenglkolceteth-3-acetat, Octyldodecylmyristat, Cetylrecinolat und Myristylmyristat.

**2.** Kosmetische Zusammensetzung nach Anspruch 1, bei der das nichtionische Tensid einen HLB-Wert von 8 - 12 aufweist.

**3.** Kosmetische Zusammensetzung nach Anspruch 1, bei der der Antiperspirantwirkstoff ausgewählt ist aus der Gruppe bestehend aus Aluminiumchlorhydrat, Aluminiumchlorid, Aluminiumsesquichlorhydrat, Zirkonylhydroxychlorid, Aluminium-Zirkoniumglycinkomplex selbst ausgewählt aus der Gruppe bestehend aus beispielsweise Aluminiumzirkonimtrichlorhydrex gly, Aluminiumzirkoniumpentachlorhydrex gly, Aluminiumzirkoniumtetrachlorhydrex gly und Aluminiumzirkoniumoctochlorhydrex gly; Aluminiumchlorhydrex PG; Aluminiumchlor hydrex PEG, Aluminiumdiculorhydrex PG, Aluminiumdichlorhydrex PEG, Aluminiumnitratohydrat und seine Kombination mit Zirkonylhydroxychloriden und -nitriden und Aluminiumzinnchlorhydraten.

**4.** Kosmetische Zusammensetzung nach Anspruch 3, bei der der Antiperspirantwirkstoff zu der kosmetischen Zusammensetzung in einer Menge im Bereich von 0,1 - 30 Gew.%, bezogen auf das Gesamtgewicht der Zusammensetzung, zugesetzt worden ist.

**5.** Kosmetische Zusammensetzung nach einem der vorhergehenden Ansprüche, bei der das als Antiperspirant wirksame Material mit einem antimikrobiellen Mittel ausgewählt aus der Gruppe bestehend aus bakteriostatischen quartären Ammoniumverbindungen, Cetyltrimethylammoniumbromid, Cetylpyridiniumchlorid, 2,4,4'-Trichlor-2'-hydroxydiphenylether, N-(4-Chlorphenyl)-N'-(3,4-dichlorphenyl)harnstoff, Silberhalogeniden, Octoxyglycerin und Zinksalzen kombiniert ist.

**6.** Kosmetische Zusammensetzung nach einem der vorhergehenden Ansprüche, bei der das vernetzte Organopolysiloxanmaterial aus mindestens einem Vernetzungsmittel und mindestens einem Vinylpolysiloxan hergestellt worden ist.

**7.** Kosmetische Zusammensetzung nach Anspruch 1, bei der das vernetzte Organopolysiloxanmaterial aus mindestens einem Vernetzungsmittel und mindestens einem alpha, omega-Dien hergestellt worden ist.

**8.** Kosmetische Zusammensetzung nach Anspruch 6, bei der Vinylpolysiloxan ausgewählt ist aus der Gruppe bestehend aus:

(a) vinylterminierten Polysiloxanen der Formel I:

$$CH_2=CH-Si(CH_3)_2-O- (Si (CH_3)_2-O)_n-Si (CH_3)_2-CH=CH_2$$

und

(b) vinylfunktionellen Copolymeren der Formel II:

$$(CH_3)_3Si-O-(Si(CH_3)_2-O-)_m-(Si (CH_3) (CH=CH_2))_n-Si(CH_3)_3,$$

in denen n eine Zahl von 1 - 100 ist und m eine Zahl von 1 - 100 ist.

**9.** Kosmetische Zusammensetzung nach Anspruch 8, bei der n eine Zahl von 10 - 50 ist.

**10.** Kosmetische Zusammensetzung nach Anspruch 8, bei der m eine Zahl von 10 - 50 ist.

**11.** Kosmetische Zusammensetzung nach Anspruch 8, bei der das Vinylpolysiloxan mindestens ein Mitglied ausgewählt aus der Gruppe bestehend aus:

(a) Polydimethylsiloxan, das monovinyl-terminiert ist,
(b) Vinylmethyl/Dimethylsiloxan-Copolymer, das trimethylsiloxy-terminiert ist,
(c) Vinylmethyl/Dimethylsiloxan-Copolymer, das vinyldimethyl-terminiert ist,
(d) Divinylmethyl-terminierten Polydimethylsiloxanen,
(e) Vinyl-Q-Harz,
(f) Vinylphenylmethyl-terminierten Dimethylsiloxanen,
(g) cyclischen Vinylmethyldimethylsiloxanen,
(h) T-Struktur-Polydimethylsiloxanen mit Vinyl am Verzweigungspunkt,
(i) T-Struktur-Polydimethylsiloxan mit Vinyl am Verzweigungsende,
(j) Diphenyldimethyl-Copolymer, das vinylterminiert ist,
(k) vinylterminierten Polymethylsiloxanen,
(l) vinylterminiertem Trifluorpropylmethylsiloxan/Dimethylsiloxan-Copolymer,
(m) vinylterminiertem Diethylsiloxancopolymer,
(n) Vinylmethylsiloxan/Dimethylsiloxan-Copolymer, das trimethylsiloxy-terminiert ist,
(o) Vinylharzen und
(p) Vinylmethylsiloxanhomopolymeren

umfaßt.

**12.** Kosmetische Zusammensetzung nach Anspruch 7, bei der das alpha,omega-Dien ausgewählt ist' aus der Gruppe bestehend aus der Formel:

$$CH_2=CH(CH_2)_xCH=CH_2,$$

in der x eine Zahl im Bereich von 1-20 ist.

**13.** Kosmetische Zusammensetzung nach Anspruch 7, bei der das alpha,omega-Dien ausgewählt ist aus der Gruppe bestehend aus 1,4-Pentadien, 1,5-Hexadien, 1,6-Heptadien, 1,7-Octadien, 1,8-Nonadien, 1,11-Dodecadien, 1,13-Tetradecadien und 1,19-Eicosadien.

**14.** Kosmetische Zusammensetzung nach Anspruch 1, bei der das Vernetzungsmittel mindestens ein Mitglied ausgewählt aus der Gruppe bestehend aus Hydrid-terminierten Polydimethylsiloxanen der Formel III ist:

$$H\text{-}Si(CH_3)_2\text{-}O\text{-}(Si(CH_3)_2\text{-}O\text{-})_p\text{-}Si(CH_3)_2\text{-}H,$$

in der p eine Zahl von 1 - 50 ist.

**15.** Kosmetische Zusammensetzung nach Anspruch 14, bei der p eine Zahl von 5 - 20 ist.

**16.** Kosmetische Zusammensetzung nach Anspruch 1, bei der das Vernetzungsmittel mindestens ein Mitglied ausgewählt aus der Gruppe bestehend aus

(a) Methylhydro-dimethylsiloxan-Copolymer mit 20 - 60 % Methylwasserstoff,
(b) Methylhydrosiloxan/Dimethylsiloxan-Copolymer,
(c) Polymethylhydrosiloxanen,
(d) Polyethylhydrosilan,
(e) Polyphenyl-(dimethylhydrosiloxy)siloxan, das Hydrid-terminiert ist,
(f) Methylhydrosiloxan/Phenylmethylsiloxan-Copolymer, das Hydrid-terminiert ist, und
(g) Methylhydrosiloxan/Octylmethylsiloxan-Copolymer ist.

**17.** Kosmetische Zusammensetzung nach Anspruch 1, bei der die flüchtigen und nichtflüchtigen Silikonmaterialien ausgewählt sind aus der Gruppe bestehend aus cyclischen und linearen flüchtigen und nichtflüchtigen Silkonen.

**18.** Kosmetische Zusammensetzung nach Anspruch 17, bei der die flüchtigen Silikone ein oder mehrere Mitglieder ausgewählt aus der Gruppe bestehend aus cyclischen Polydimethylsiloxanen mit der Formel IV:

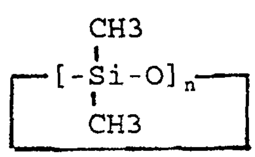

sind, in der n eine Zahl mit einem Wert von 3 - 7 ist.

**19.** Kosmetische Zusammensetzung nach Anspruch 17, bei der die nichtflüchtigen und flüchtigen linearen Silikone ein oder mehrere Mitglieder ausgewählt aus der Gruppe bestehend aus linearen Polydimethylsiloxanen mit der Formel V:

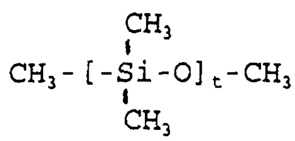

sind, in der t so gewählt ist, daß das Molekulargewicht im Bereich von 800 - 260 000 liegt und die Viskosität im Bereich von 0,05 - 6000 $cm_2/s$ (5 - 600 000 Centistoke) liegt.

**20.** Kosmetische Zusammensetzung nach Anspruch 1, bei der das nichtionische Tensid mindestens ein Mitglied ausgewählt aus der Gruppe bestehend aus:

(a) Sorbitanestern und ethoxylierten Sorbitanestern,
(b) Ethoxylaten,
(c) ethoxylierten Addukten,
(e) Propoxylaten,
(f) ethoxylierten modifzierten Triglyceriden,
(g) alkylphenolaromatischen Ethoxylaten,
(h) Block-Copolymeren, die alkoxylierte Glykole sind und ethoxylierte und propoxylierte Segmente aufweisen,
(i) Silikonpolyethern ist.

**21.** Kosmetische Zusammensetzung nach Anspruch 20, bei der

(a) die Sorbintanester und ethoxylierten Sorbitanester ausgewählt sind aus der Gruppe bestehend aus PEG-20-Sorbitanisostearat, Sorbitanmonolaurat, Polysorbat-40, Polysorbat-60, Polysorbat-80 und Polysorbat-120,

(b) die Ethoxylate ausgewählt sind aus der Gruppe bestehend aus Ceteth-20, PEG-30-Castoröl, PEG-40-hydriertes Castoröl, PEG-60-hydriertes Castoröl, Laureth-7, Isolaureth-6, Steareth-10, Steareth-20, Steareth-21. Ceteareth-12, Ceteareth-20, Oleth-5, Oleth-9, Oleth-10, Oleth-20 und PPG-3-Buteth-5,

(c) die ethoxylierten Addukte ausgewählt sind aus der Gruppe bestehend aus PEG-25-Stearat, Glycerylstearat und PEG-100-Stearat,

(e) die Propoxylate ausgewählt sind aus der Gruppe bestehend aus PPG-10-Butandiol, PPG-1-PEG-9-Laurylglykolether, PPG-3-Deceth-3 und PPG-5-Ceteth-20,

(f) die ethoxylierten modifizierten Triglyceride ausgewählt sind aus der Gruppe bestehend aus PEG-20-Maisglyceriden und PEG-12-Palmkernglyceriden,

(g) die alkylphenolaromatischen Ethoxylate ausgewählt sind aus der Gruppe bestehend aus Dinonylphenol ethoxyliert mit 9 Molen Ethylenoxid, Octylphenol ethoxyliert mit 20 Molen Ethylenoxid und Octylphenol ethoxyliert mit 40 Molen Ethylenoxid,

(h) die Block-Copolymeren ausgewählt sind aus der Gruppe bestehend aus alkoxylierten Glykolen mit ethoxylierten und propoxylierten Segmenten, Poloxamer 182 und Poloxamer 234,

(i) die Silikonpolyether ausgewählt sind aus der Gruppe bestehend aus (1) Dimethiconcopolyolen, (2) Dimethiconcopolyollauraten, (3) Dimethiconcopolyolisostearaten, (4) Polydimethiconcopolyolen und (5) Eugenodimethiconcopolyolen.

**22.** Kosmetische Zusammensetzung nach Anspruch 1, die hergestellt worden ist, indem das Silikongelmaterial mit 1 - 30 Gew.% von mindestens einem vernetzten Organopolysiloxanmaterial und 70 - 99 Gew.% des Trägers, bezogen auf das Gesamtgewicht des Silikongelmaterials, gebildet worden ist.

**23.** Kosmetische Zusammensetzung nach Anspruch 1, die mit 0,1 - 20% Tensid, bezogen auf das Gesamtgewicht der Zusammensetzung, hergestellt worden ist.

**24.** Kosmetische Zusammensetzung nach Anspruch 1, die hergestellt worden ist, indem 10 - 80% Silikongelmaterial, 0,1 - 30% Antiperspirantwirkstoff, 0,1 - 20% Tensid kombiniert worden sind.

**25.** Kosmetische Zusammensetzung nach Anspruch 24, bei der der Träger hergestellt worden ist, indem mindestens ein Silikonmaterial mit 0,5 - 50% von mindestens einem Aufweichmaterial kombiniert worden ist.

**26.** Kosmetische Zusammensetzung nach Anspruch 25, bei der die Zusammensetzung mindestens einen Verdicker enthält.

## Revendications

1. Composition cosmétique antitranspirante et/ou déodorante préparée en combinant:

   (a) au moins une substance active antitranspirante ;
   (b) un gel de silicone préparée en combinant :

      (i) un organo-polysiloxane réticulé comme agent gélifiant dans lequel l'organo-polysiloxane est préparée à partir d'au moins un agent de réticulation d'hydrure de silicone et d'au moins un élément choisi dans le groupe composé de (A) un polysiloxane vinylique et de (B) un diène alpha oméga ; et
      (ii) un excipient de base liquide pour l'agent gélifiant dans lequel l'excipient est préparé en combinant au moins un élément du groupe composé de substances siliconées volatiles et de substances siliconées non volatiles, et facultativement dans lequel au moins un émollient liquide qui est soluble ou miscible dans la (ou les) substance(s) siliconée(s) choisie(s) est ajouté pour former l'excipient ;

   (c) au moins un agent tensio-actif non ionique ayant une valeur HLB de 8-16 ; et
   (d) au moins une matière émolliente choisie dans le groupe composé de myristate d'isopropyle, de palmipate d'isopropyle, de stéarate d'isopropyle, d'isostéarate d'isopropyle, de stéarate de butyle, de stéarate d'octyle, de laurate d'hexyle, de stéarate de cétyle, d'adipate de diisopropyle, d'oléate isodécylique, de sébaçate de diisopropyle, de lactate d'isostéaryle, de benzoate d'alkyle $C_{12-15}$, de myreth-3-myristate, de malate de dioctyle, de diheptanoate de néopentyl glycol, de dioctanoate de néopentyl glycol, de dibenzoate de dipropylène glycol, de lactate d'alcools $C_{12-15}$, de décanoate d'isohexyle, de caprate d'isohexyle, de dioctanoate de diéthylène glycol, d'isononanoate d'octyle, d'octanoate isodécylique, de diisononanoate de diéthylène glycol, d'isononanoate d'isononyle, d'isostéarate d'isostéaryle, de béhénate de béhényle, de fumarate d'alkyle $C_{12-15}$, de benzoate de laureth-2, d'acétate d'isoceteth-3 de propylène glycol, d'acétate ceteth-3 de propylène glycol, de myristate d'octyldodécyle, de ricinoléate de cétyle et de myristate myristique.

2. Composition cosmétique selon la revendication 1 dans laquelle l'agent tensio-actif non ionique a une valeur HLB de 8-12.

3. Composition cosmétique selon la revendication 1 dans laquelle la substance active antitranspirante est choisie dans le groupe composé de chlorohydrate d'aluminium, de chlorure d'aluminium, de sesquichlorohydrate d'aluminium ; d'hydroxychlorures de zirconyle; d'un complexe aluminium-zirconium avec la glycine lui,même choisi dans le groupe composé, par exemple, d'aluminium zirconium trichlorohydrex gly, d'aluminium zirconium pentachlorohydrex gly, d'aluminium zirconium tétrachlorohydrex gly et d'aluminium zirconium octochlorohydrex gly; de PG aluminium chlorohydrex ; de PEG aluminium chlorohydrex ; de PG aluminium dichlorohydrex ; de PEG aluminium de dichlorohydrex ; de nitratohydrate d'aluminium et sa combinaison avec les hydroxychlorures de zirconyle et les nitrures; et de chlorohydrates d'aluminium stanneux.

4. Composition cosmétique selon la revendication 3 dans laquelle la substance active antitranspirante est ajoutée à la composition cosmétique en quantité comprise entre 0,1 et 30 pour cent en poids sur la base du poids total de la composition.

5. Composition cosmétique selon l'une quelconque des revendications précédentes, dans laquelle la substance active antitranspirante est combinée avec un agent antimicrobien choisi dans le groupe composé des composés quaternaires d'ammonium bactéricides, du bromure de cétyltriméthylammonium, du chlorure de cétyl pyridinium, du 2,4,4'-trichloro-2'-hydroxydiphenylether, de l'urée N-(4-chlorophényle)-N'-(3,4-dichlorophényle), des halogénures d'argent, de l'octoxyglycérine, et des sels de zinc.

6. Composition cosmétique selon l'une quelconque des revendications précédentes, dans laquelle l'organo-polysiloxane réticulé est préparé à partir d'au moins un agent de réticulation et d'au moins un polysiloxane vinylique.

7. Composition cosmétique selon la revendication 1 dans laquelle l'organo-polysiloxane réticulé est préparé à partir d'au moins un agent de réticulation et d'au moins un diène alpha, oméga.

8. Composition cosmétique selon la revendication 6, dans laquelle le polysiloxane vinylique est choisi dans le groupe composé :

(a) des polysiloxanes à groupement terminal vinyl de Formule I :

$$CH_2=CH\text{-}Si(CH_3)_2\text{-}O\text{-}(\text{-}Si(CH_3)_2\text{-}O\text{-})_n\text{-}Si(CH_3)_2\text{-}CH=CH_2 \qquad \text{Formule I}$$

et (b) des copolymères fonctionnels vinyliques de Formule II :

$$(CH_3)_3\ Si\text{-}O\text{-}(Si(CH_3)_2\text{-}O\text{-})_m\text{-}(Si(CH_3)(CHCH_2))_n\ \text{-}Si(CH_3)_3 \qquad \text{Formule II}$$

où n = un nombre de 1 à 100 et m = un nombre de 1 à 100.

**9.** Composition cosmétique selon la revendication 8 dans laquelle n = un nombre de 10 à 50.

**10.** Composition cosmétique selon la revendication 8 dans laquelle m = un nombre de 10 à 50.

**11.** Composition cosmétique selon la revendication 8 dans laquelle le polysiloxane vinylique comprend au moins un élément choisi dans le groupe composé de :

(a) polydiméthylsiloxane, à groupement terminal monovinyl ;
(b) copolymère de diméthylsiloxane, vinyl méthyle à groupement terminal triméthylsiloxy ;
(c) copolymère de diméthylsiloxane, vinyl méthyle, à groupement terminal vinyl diméthyle;
(d) polydiméthylsiloxanes à groupement terminal divinyl méthyle ;
(e) résine Q vinylique ;
(f) diméthylsiloxanes à groupement terminal vinyl phényl méthyle ;
(g) diméthylsiloxanes vinyl méthyle cycliques;
(h) polydiméthylsiloxanes à structure ramifiée avec vinyl à l'embranchement ;
(i) polydiméthylsiloxane à structure ramifiée avec vinyl en bout de branche ;
(j) copolymère de diphényle diméthyle à groupement terminal vinyl;
(k) polyméthylsiloxanes à groupement terminal vinyl;
(l) copolymère diméthylsiloxane - méthylsiloxane trifluoropropylique à groupement terminal vinyl ;
(m) copolymère diéthylsiloxane à groupement terminal vinyl ;
(n) copolymère diméthylsiloxane - siloxane vinyl méthyle à groupement terminal triméthylsiloxy ;
(o) gommes vinyliques ; et
(p) homopolymères de siloxane vinyl méthyle.

**12.** Composition cosmétique selon la revendication 7 dans laquelle le diène alpha oméga, est choisi dans le groupe comprenant la Formule :

$$CH_2=CH(CH_2)_xCH=CH_2,$$

où x est un nombre compris entre 1 et 20.

**13.** Composition cosmétique selon la revendication 7 dans laquelle le diène alpha oméga, est choisi dans le groupe composé de pentadiène-1,4; d'hexadiène- 1,5 ; d'heptadiène-1,6 ; d'octadiène-1,7 ; de nonadiène-1,8 ; de dodécadiène-1,11 ; de tétradécadiène-1,13; et d'éicosadiène-1,19.

**14.** Composition cosmétique selon la revendication 1 dans laquelle l'agent de réticulation est au moins un élément choisi dans le groupe composé de polydiméthylsiloxanes à groupement terminal hydrure de Formule III :

$$H\text{-}Si(CH_3)_2\text{-}O\text{-}(Si(CH_3)_2\text{-}O\text{-})_p\text{-}Si(CH_3)_2\text{-}H \qquad \text{Formule III}$$

où p = un nombre de 1 à 50.

**15.** Composition cosmétique selon la revendication 14 dans laquelle p = un nombre de 5 à 20.

**16.** Composition cosmétique selon la revendication 1 dans laquelle l'agent de réticulation est au moins un élément choisi dans le groupe composé de :

(a) copolymère de diméthylsiloxane méthylé et hydrogéné avec 20-60 % d'hydrométhyle ;
(b) copolymère diméthylsiloxane -méthylhydrosiloxane ;
(c ) polyméthylhydrosiloxanes;
(d) polyéthylhydrosilane ;
(e) polyphényl-(diméthylhydrosiloxy) siloxane à groupement terminal hybride ;
(f) copolymère siloxane méthyl phényle-méthylhydrosiloxane à groupement terminal hybride.
(g) copolymère octylméthylsiloxane -méthylhydrosiloxane.

**17.** Composition cosmétique selon la revendication 1 dans laquelle les substances siliconées volatiles et non volatiles sont choisies dans le groupe composé de substances siliconées volatiles et non volatiles cycliques et linéaires ;

**18.** Composition cosmétique selon la revendication 17 dans laquelle les substances siliconées volatiles sont un ou plusieurs éléments choisis dans le groupe composé de polydiméthylsiloxanes cycliques représentés par la Formule IV :

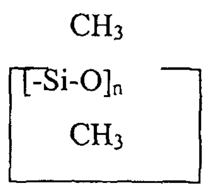

Formule IV

où n est un nombre entier ayant une valeur de 3-7.

**19.** Composition cosmétique selon la revendication 17 dans laquelle les substances siliconées linéaires volatiles et non-volatiles sont un ou plusieurs éléments choisis dans le composé de polydiméthylsiloxanes linéaires représentés par la Formule V :

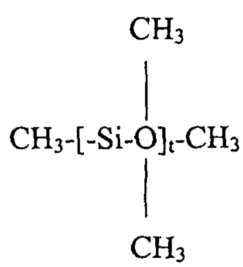

Formule V

dans laquelle t est choisi de sorte que le poids moléculaire est compris entre 800 et 260000 et la viscosité est comprise entre 0,05 et 6000 $cm^2/s$ (5-600000 centistokes).

**20.** Composition cosmétique selon la revendication 1 dans laquelle l'agent tensio-actif non ionique est au moins un élément choisi dans le groupe composé de :

(a) esters de sorbitan et esters éthoxylés de sorbitan;
(b) éthoxylates ;
(c) adducts éthoxylés ;
(e) propoxylates ;
(f) triglycérides éthoxylés modifiés ;
(g) éthoxylates d'alkylphénol aromatiques ;
(h) copolymères blocs qui sont des glycols alcoxylés ayant des segments propoxylés et éthoxylés ;
(i) polyéthers de silicone.

**21.** Composition cosmétique selon la revendication 20 dans laquelle

(a) les esters de sorbitan et les esters éthoxylés de sorbitan sont choisis dans le groupe composé de PEG-20 isostéarate de sorbitan, de monolaurate de sorbitan, de polysorbate 40, de polysorbate 60, de polysorbate 80 et de polysorbate 120 ;
(b) les éthoxylates sont choisis dans le groupe composé de Ceteth-20, de PEG-30 huile de ricin, de PEG-40 huile de ricin hydrogénée, de PEG-60 huile de ricin hydrogénée, de Laureth-7, d'Isolaureth-6, de Stéareth-10, de Stéareth-20, de Stéareth-21, de Cétéareth-12, de Cétéareth-20, d'Oleth-5, d'Oleth-9, d'Oleth-10, d'Oleth-20 et de PPG-3-Buteth-5 ;
(c) les adducts éthoxylés sont choisis dans le groupe composé de PEG-25 stéarate, de stéarate de glycéryle et de PEG-100 stéarate ;
(e) les propoxylates sont choisis dans le groupe composé de PPG-10 butanédiol, d'Ether de Glycol et de Lauryle PPG-1-PEG-9, de PPG-3-déceth-3, et de PPG-5-céteth-20 ;
(f) les triglycérides éthoxylés modifiés sont choisis dans le groupe composé de PEG-20 glycérides de maïs et de PEG-12 glycérides de palmiste;
(g) les éthoxylates d'alkylphénol aromatiques sont choisis dans le groupe composé de dinonylphénol ethoxylé avec 9 moles d'oxyde d'éthylène, d'octylphénol éthoxylé avec 20 moles d'oxyde d'éthylène et d'octyphénol éthoxylé avec 40 moles d'oxyde d'éthylène ;
(h) les copolymères blocs sont choisis dans le groupe composé de glycols alcoxylés ayant des segments propoxylés et éthoxylé, de Poloxamère 182 et de Poloxamère 234 ;
(i) les polyéthers de silicone sont choisis dans le groupe comprenant (1) des diméthicones copolyols ; (2) des laurates de diméthicone copolyol ; (3) des isostéarates de diméthicone copolyol ; (4) des polydiméthicones copolyols ; et (5) des eugenodiméthicones copolyols.

**22.** Composition cosmétique selon la revendication 1 préparée en formant le gel de silicone avec 1-30 % en poids d'au moins un organo-polysiloxane réticulé et 70-99 % en poids de l'excipient, sur la base du poids total du gel de silicone.

**23.** Composition cosmétique selon la revendication 1 préparée avec 0,1-20 % de l'agent tensio-actif sur la base du poids total de la composition.

**24.** Composition cosmétique selon la revendication 1 préparée en combinant 10-80 % de gel de silicone ; 0,1-30 % de substance active antitranspirante ; 0,1-20 % de l'agent tensio-actif.

**25.** Composition cosmétique selon la revendication 24 dans laquelle l'excipient est préparée en combinant au moins une substance siliconée avec 0,5-50 % d'au moins un émollient.

**26.** Composition cosmétique selon la revendication 25 dans laquelle la composition contient au moins un épaississant.